# EUROPEAN PATENT SPECIFICATION

(11) **EP 4 132 924 B1**
(45) Date of publication and mention of the grant of the patent: **21.02.2024**
(21) Application number: 21716439.1
(22) Date of filing: 06.04.2021
(51) Int. Cl.: C07D 413/04, A01N 43/86

(54) **MICROBIOCIDAL QUINOLINE DIHYDRO-(THIAZINE)OXAZINE DERIVATIVES**
MIKROBIOZIDE CHINOLINDIHYDRO-(THIAZINE)OXAZIN-DERIVATE
DÉRIVÉS D'OXAZINE(THIAZINE) DIHYDROQUINOLINE MICROBIOCIDE

(30) Priority: 08.04.2020 EP 20168704
(43) Date of publication of application: 15.02.2023
(73) Proprietor: Syngenta Crop Protection AG, 4058 Basel (CH)
(72) Inventor: BOU HAMDAN, Farhan, 4332 Stein (CH); QUARANTA, Laura, 4058 Basel (CH); WILLIAMS, Simon, 4332 Stein (CH); GERMAIN, Nicolas, 4332 Stein (CH)
(74) Representative: SYNGENTA IP
(86) International application number: PCT/EP2021/058969
(87) International publication number: WO 2021/204822

(56) References cited:
- EP-A1- 1 736 471
- EP-A1- 2 280 009
- EP-A1- 2 517 562
- WO-A1-2016/156129
- WO-A1-2017/153380
- WO-A1-2018/073110
- WO-A1-2019/053015

## Description

The present invention relates to microbiocidal quinoline dihydro-(thiazine)oxazine derivatives, e.g. as active ingredients, which have microbiocidal activity, in particular fungicidal activity. The invention also relates to preparation of these quinoline dihydro-(thiazine)oxazine derivatives, to intermediates useful in the preparation of these quinoline dihydro-(thiazine)oxazine derivatives, to the preparation of these intermediates, to agrochemical compositions which comprise at least one of the quinoline dihydro-(thiazine)oxazine derivatives, to preparation of these compositions and to the use of the quinoline dihydro-(thiazine)oxazine derivatives or compositions in agriculture or horticulture for controlling or preventing infestation of plants, harvested food crops, seeds or non-living materials by phytopathogenic microorganisms, in particular fungi.

Certain fungicidal quinoline compounds are described in WO 2005/070917, WO 2011/077514, WO 2016/156129, WO 2018/073110, WO 2019/053015 and WO 2017/153380.

According to the present invention, there is provided a compound of formula (I): 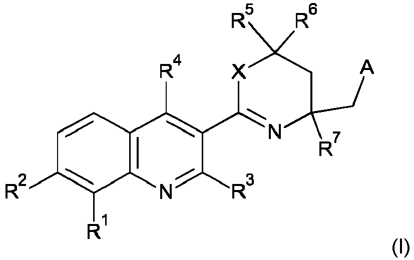 wherein:
X is O or S;
R¹ is halogen, methyl or cyano;
R² is hydrogen or halogen;
R³ and R⁴ are each independently hydrogen or methyl;
R⁵ and R⁶ are each independently selected from hydrogen, C₁-C₅alkyl, C₁-C₅haloalkyl, cyanoC₁₋C₅alkyl, C₁-C₃alkoxyC₁-C₅alkyl, C₁-C₃alkylthioC₁-C₅alkyl, C₂-C₅alkenyl, C₂-C₅haloalkenyl, cyanoC₂-C₅alkenyl, C₁-C₃alkoxyC₂-C₅alkenyl, C₁-C₃alkylthioC₂-C₅alkenyl, C₂-C₅alkynyl, C₂-C₅haloalkynyl, cyanoC₂-C₅alkynyl, C₁-C₃alkoxyC₂-C₅alkynyl, C₁-C₃alkylthioC₂-C₅alkynyl and C₃-C₆cycloalkyl, wherein cycloalkyl is optionally substituted with 1, 2 or 3 substituents independently selected from halogen, cyano, C₁-C₃alkyl, C₁-C₃alkoxy and C₁-C₃alkylthio; or
R⁵ and R⁶ together with the carbon atom to which they are attached represent C₃-C₆cycloalkyl, wherein the cycloalkyl group is optionally substituted with 1, 2 or 3 substituents independently selected from halogen, cyano and C₁-C₃alkyl;
R⁷ is hydrogen, C₁-C₄alkyl or C₃-C₄cycloalkyl;
A is C₁-C₅alkyl, C₂-C₅alkenyl, C₃-C₆cycloalkyl, C₄₋C₆cycloalkenyl, C₁-C₅haloalkyl, C₂-Cshaloalkenyl, C₁-C₄alkoxy, Cs-Csalkenyloxy, Cs-Csalkynyloxy, C₁-C₄haloalkoxy, C₃-C₆cycloalkoxy and phenyloxy, wherein the C₃-C₆cycloalkoxy and phenyloxy is optionally substituted with 1, 2 or 3 substituents independently selected from R⁸;
R⁸ is halogen, nitro, cyano, C₁-C₅alkyl, C₁-C₂haloalkyl, C₁-C₅alkoxy, Cs-Csalkenyloxy, C₃-Csalkynyloxy and C₁-C₅alkylthio; or
an agronomically acceptable salt, an N-oxide and/or S-oxide or a stereoisomer thereof.

Surprisingly, it has been found that the novel compounds of Formula (I) have, for practical purposes, a very advantageous level of biological activity for protecting plants against diseases that are caused by fungi.

According to a second aspect of the invention, there is provided an agrochemical composition comprising a fungicidally effective amount of a compound of Formula (I). Such an agricultural composition may further comprise at least one additional active ingredient and/or an agrochemically-acceptable diluent or carrier.

According to a third aspect of the invention, there is provided a method of controlling or preventing infestation of useful plants by phytopathogenic microorganisms, wherein a fungicidally effective amount of a compound of Formula (I), or a composition comprising this compound as active ingredient, is applied to the plants, to parts thereof or the locus thereof.

According to a fourth aspect of the invention, there is provided the use of a compound of Formula (I) as a fungicide. According to this particular aspect of the invention, the use excludes methods for the treatment of the human or animal body by surgery or therapy.

Where substituents are indicated as being optionally substituted, this means that they may or may not carry one or more identical or different substituents, e.g. one to four substituents. Normally not more than three such optional substituents are present at the same time. Preferably not more than two such optional substituents are present at the same time (i.e. the group may be optionally substituted by one or two of the substituents indicated as "optional"). Where the "optional substituent" group is a larger group, such as cycloalkyl or phenyl, it is most preferred that only one such optional substituent is present. Where a group is indicated as being substituted, e.g. alkyl, this includes those groups that are part of other groups, e.g. the alkyl in alkylthio.

As used herein, the term "halogen" or "halo" refers to fluorine (fluoro), chlorine (chloro), bromine (bromo) or iodine (iodo), preferably fluorine, chlorine or bromine.

As used herein, cyano means a -CN group.

As used herein, nitro means an -NO₂ group.

As used herein, the term "C₁₋C₅alkyl" refers to a straight or branched hydrocarbon chain radical consisting solely of carbon and hydrogen atoms, containing no unsaturation, having from one to five carbon atoms, and which is attached to the rest of the molecule by a single bond. C₁₋₃alkyl and C₁₋₂alkyl are to be construed accordingly. Examples of C₁₋C₅alkyl include, but are not limited to, methyl, ethyl, *n-*propyl, 1-methylethyl (iso-propyl), n-butyl, and 1,1-dimethylethyl (t-butyl). A "C₁₋C₅alkylene" group refers to the corresponding definition of C₁-C₅alkyl, except that such radical is attached to the rest of the molecule by two single bonds. Examples of C₁₋C₅alkylene, are -CH₂- and -CH₂CH₂-.

As used herein, the term "C₁₋C₅haloalkyl" refers to a C₁₋C₅alkyl radical as generally defined above substituted by one or more of the same or different halogen atoms. C₁₋C₂haloalkyl is to be construed accordingly. Examples of C₁₋C₅haloalkyl include, but are not limited to fluoromethyl, fluoroethyl, difluoromethyl, trifluoromethyl, and 2,2,2-trifluoroethyl.

As used herein, the term "cyanoC₁₋C₅alkyl" refers to a C₁-C₅alkyl radical as generally defined above substituted by one or more cyano groups. Examples of cyanoC₁-C₅alkyl include, but are not limited to cyanomethyl.

As used herein, the term "C₁₋C₃alkoxyC₁₋C₅alkyl" refers to a radical of the formula R_{b}-O-Rₐ- where R_{b} is a C₁₋C₃alkyl radical as generally defined above, and Rₐ is a C₁₋C₅alkylene radical as generally defined above.

As used herein, the term "C₁₋C₃alkylthioC₁-C₅alkyl" refers to a radical of the formula R_{b}-S-Rₐ-where R_{b} is a C₁₋C₃alkyl radical as generally defined above, and Rₐ is a C₁₋C₅alkylene radical as generally defined above.

As used herein, the term "C₂₋C₅alkenyl" refers to a straight or branched hydrocarbon chain radical group consisting solely of carbon and hydrogen atoms, containing at least one double bond that can be of either the (E)- or (Z)-configuration, having from two to five carbon atoms, which is attached to the rest of the molecule by a single bond. C₃₋C₅alkenyl is to be construed accordingly. Examples of C₂₋C₅alkenyl include, but are not limited to, vinyl (ethenyl), prop-1-enyl, allyl (prop-2-enyl), and but-1-enyl. A "C₂₋Csalkenylene" group refers to the corresponding definition of C₂₋C₅alkenyl, except that such radical is attached to the rest of the molecule by two single bonds. Examples of C₂₋C₅alkenylene, are -CH=CH₂-and -CH₂CH=CH₂-.

As used herein, the term "C₂₋C₅haloalkenyl" refers to a C₂₋C₅alkenyl radical as generally defined above substituted by one or more of the same or different halogen atoms.

As used herein, the term cyanoC₂-C₅alkenyl refers to a C₂₋C₅alkenyl radical as generally defined above substituted by one or more cyano groups.

As used herein, the term "C₁₋C₃alkoxyC₂₋C₅alkenyl" refers to a radical of the formula R_{b}-O-Rₐ-where R_{b} is a C₁₋C₃alkyl radical as generally defined above, and Rₐ is a C₂₋C₅alkyenlene radical as generally defined above.

As used herein, the term "C₁₋C₃alkylthioC₂₋C₅alkenyl" refers to a radical of the formula R_{b}-S-Rₐ-where R_{b} is a C₁₋C₃alkyl radical as generally defined above, and Rₐ is a C₂₋C₅alkenylene radical as generally defined above.

As used herein, the term "C₂₋C₅alkynyl" refers to a straight or branched hydrocarbon chain radical group consisting solely of carbon and hydrogen atoms, containing at least one triple bond, having from two to five carbon atoms, and which is attached to the rest of the molecule by a single bond. The term "C₃₋C₅alkynyl" is to be construed accordingly. Examples of C₂₋C₅alkynyl include, but are not limited to, ethynyl, prop-1-ynyl, propargyl (prop-2-ynyl), and but-1-ynyl. A "C₂₋C₅alkynylene" group refers to the corresponding definition of C₂₋C₅alkynyl, except that such radical is attached to the rest of the molecule by two single bonds. Examples of C₂₋C₅alkynylene, are -C=CH- and -CH₂C≡CH-.

As used herein, the term "C₂₋C₅haloalkynyl" refers to a C₂₋C₅alkynyl radical as generally defined above substituted by one or more of the same or different halogen atoms.

As used herein, the term cyanoC₂-C₅alkynyl refers to a C₂₋C₅alkynyl radical as generally defined above substituted by one or more cyano groups.

As used herein, the term "C₁₋C₃alkoxyC₂₋C₅alkynyl" refers to a radical of the formula R_{b}-O-Rₐ-where R_{b} is a C₁₋C₃alkyl radical as generally defined above, and Rₐ is a C₂₋C₅alkynlene radical as generally defined above.

As used herein, the term "C₁₋C₃alkylthioC₂₋C₅alkynyl" refers to a radical of the formula R_{b}-S-Rₐ-where R_{b} is a C₁₋C₃alkyl radical as generally defined above, and Rₐ is a C₂₋C₅alkynylene radical as generally defined above.

As used herein, the term "C₃₋C₆cycloalkyl" refers to a stable, monocyclic ring radical which is saturated and contains 3 to 6 carbon atoms. C₃₋C₄cycloalkyl is to be construed accordingly. Examples of C₃₋C₆cycloalkyl include, but are not limited to, cyclopropyl, cyclobutyl, cyclopentyl and cyclohexyl.

As used herein, the term "C₁₋C₃alkoxy" refers to a radical of the formula RₐO- where Rₐ is a C₁₋C₃alkyl radical as generally defined above. Examples of C₁-C₃alkoxy include, but are not limited to, methoxy, ethoxy, n-propoxy, iso-propoxy.

As used herein, the term "C₃₋C₅alkenyloxy" refers to a radical of the formula RₐO- where Rₐ is a C₃₋C₅alkenyl radical as generally defined above.

As used herein, the term "C₃₋C₅alkynyloxy" refers to a radical of the formula RₐO- where Rₐ is a C₃₋C₅alkynyl radical as generally defined above.

As used herein, the term C₁-C₄haloalkoxy refers to a C₁₋C₄alkoxy radical as generally defined above substituted by one or more of the same or different halogen atoms.

As used herein, the term C₃-C₆cycloalkoxy refers to a radical of the formula RₐO-, where Rₐ is a C₃₋C₆cycloalkyl radical as generally defined above.

As used herein, the term "C₁₋C₃alkylthio" refers to a radical of the formula RₐS- where Rₐ is a C₁₋C₃alkyl radical as generally defined above.

As used herein, the term "C₄₋C₆cycloalkenyl" refers to a stable, monocyclic ring radical which is partially unsaturated and contains 4 to 6 carbon atoms.

The presence of one or more possible asymmetric carbon atoms in a compound of formula (I) means that the compounds may occur in optically isomeric forms, i.e. enantiomeric or diastereomeric forms. Also atropisomers may occur as a result of restricted rotation about a single bond. Formula (I) is intended to include all those possible isomeric forms and mixtures thereof. The present invention includes all those possible isomeric forms and mixtures thereof for a compound of formula (I). Likewise, formula (I) is intended to include all possible tautomers. The present invention includes all possible tautomeric forms for a compound of formula (I).

In each case, the compounds of formula (I) according to the invention are in free form, in oxidized form as a N-oxide, in covalently hydrated form, or in salt form, e.g., an agronomically usable or agrochemically acceptable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The following list provides definitions, including preferred definitions, for substituents X, R¹, R², R³, R⁴, R⁵, R⁶, R⁷, A and R⁸ with reference to the compounds of Formula (I) according to the invention. For any one of these substituents, any of the definitions given below may be combined with any definition of any other substituent given below or elsewhere in this document.

X is O or S. In some embodiments of the invention, X is O. In other embodiments of the invention, X is S.

R¹ is halogen, methyl or cyano. Preferably, R¹ is fluoro or chloro, and most preferably, fluoro.

R² is hydrogen or halogen. Preferably, R² is hydrogen or fluro.

R³ and R⁴ are each independently hydrogen or methyl. Preferably, R³ is methyl and R⁴ is hydrogen, R³ is hydrogen and R⁴ is methyl, or R³ and R⁴ are hydrogen. Most preferably, R³ and R⁴ are hydrogen.

R⁵ and R⁶ are each independently selected from hydrogen, C₁-C₅alkyl, C₁-C₅haloalkyl, cyanoC₁₋Csalkyl, C₁-C₃alkoxyC₁-C₅alkyl, C₁-C₃alkylthioC₁-C₅alkyl, C₂-C₅alkenyl, C₂-C₅haloalkenyl, cyanoC₂-Csalkenyl, C₁-C₃alkoxyC₂-C₅alkenyl, C₁-C₃alkylthioC₂-C₅alkenyl, C₂-C₅alkynyl, C₂-C₅haloalkynyl, cyanoC₂-C₅alkynyl, C₁-C₃alkoxyC₂-C₅alkynyl, C₁-C₃alkylthioC₂-C₅alkynyl and C₃-C₆cycloalkyl, wherein cycloalkyl is optionally substituted with 1, 2 or 3 substituents independently selected from halogen, cyano, C₁-C₃alkyl, C₁-C₃alkoxy and C₁-C₃alkylthio, or R⁵ and R⁶ together with the carbon atom to which they are attached represent C₃-C₆cycloalkyl, wherein the cycloalkyl group is optionally substituted with 1, 2 or 3 substituents independently selected from halogen, cyano and C₁-C₃alkyl.

Preferably, R⁵ and R⁶ are each independently selected from hydrogen, C₁-C₄alkyl, C₁-C₂fluoroalkyl, cyanoC₁₋C₂alkyl, C₁-C₂alkoxyC₁-C₂alkyl, C₁-C₂alkylthioC₁-C₂alkyl, C₂-C₄alkenyl, C₂-C₄fluoroalkenyl, cyanoC₂-C₄alkenyl, C₁-C₂alkoxyC₂-C₄alkenyl, C₁-C₂alkylthioC₂-C₄alkenyl, C₂-C₄alkynyl, C₂-C₄fluoroalkynyl, cyanoC₂-C₄alkynyl, C₁-C₂alkoxyC₂-C₄alkynyl, C₁-C₂alkylthioC₂-C₄alkynyl and C₃-C₆cycloalkyl, wherein cycloalkyl is optionally substituted with 1, 2 or 3 substituents independently selected from halogen, cyano, C₁-C₃alkyl, C₁-C₃alkoxy and C₁-C₃alkylthio.

More preferably, R⁵ and R⁶ are each independently selected from hydrogen or C₁-C₅alkyl. Still more preferably, R⁵ and R⁶ are each independently selected from hydrogen, methyl or ethyl. Most preferably, R⁵ and R⁶ are methyl. Alternatively, R⁵ and R⁶ together form a -(CH₂)ₙ- group bound to the carbon atom to which they are attached, wherein n is 2, 3, 4 or 5.

R⁷ is hydrogen, C₁-C₄alkyl or C₃-C₄cycloalkyl. Preferably, R⁷ is hydrogen or C₁-C₄alkyl. More preferably, R⁷ is hydrogen, methyl, ethyl or iso-propyl.

A is C₁-C₅alkyl, C₂-C₅alkenyl, C₃-C₆cycloalkyl, C₄₋C₆cycloalkenyl, C₁-C₅haloalkyl, C₂-Cshaloalkenyl, C₁-C₄alkoxy, Cs-Csalkenyloxy, Cs-Csalkynyloxy, C₁-C₄haloalkoxy, C₃-C₆cycloalkoxy and phenyloxy, wherein the C₃-C₆cycloalkoxy and phenyloxy is optionally substituted with 1, 2 or 3 substituents independently selected from R⁸, wherein R⁸ is halogen, nitro, cyano, C₁-C₅alkyl, C₁-C₂haloalkyl, C₁-C₅alkoxy, Cs-Csalkenyloxy, C₃-C₅alkynyloxy and C₁-C₅alkylthio.

Preferably, A is C₁-C₅alkyl, C₂-C₅alkenyl, C₃-C₆cycloalkyl, C₄₋C₆cycloalkenyl, C₁-C₄haloalkyl, C₂-C₅haloalkenyl, C₃-C₆cycloalkoxy and phenyloxy, wherein the C₃-C₆cycloalkoxy and phenyloxy is optionally substituted with 1 or 2 substituents independently selected from R⁸, wherein R⁸ is halogen, nitro, cyano, methyl, ethyl, trifluoromethyl, 2,2,2-trifluoroethyl, methoxy, ethoxy, Cs-Csalkenyloxy, C₃-C₅alkynyloxy and C₁-C₃alkylthio.

More preferably, A is C₁-C₄alkyl, C₂-C₅alkenyl, C₃-C₆cycloalkyl, C₄₋C₆cycloalkenyl, C₁-C₄fluoroalkyl, C₂-C₅fluoroalkenyl, C₂-C₅chloroalkenyl, C₃-C₆cycloalkoxy and phenyloxy, wherein the phenyloxy is optionally substituted with 1 or 2 substituents independently selected from R⁸, wherein R⁸ is fluoro, chloro, bromo, nitro, cyano, methyl, ethyl, trifluoromethyl, methoxy and ethoxy.

Selected A groups may include: 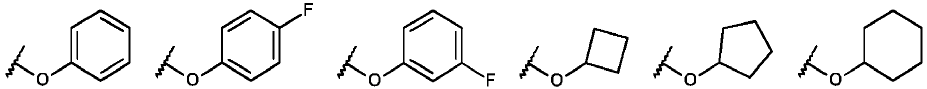 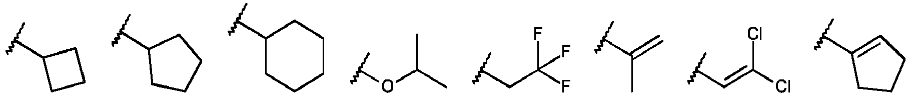 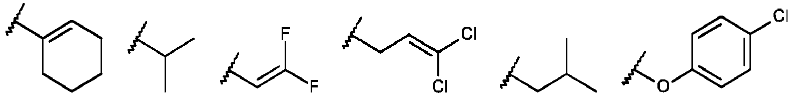 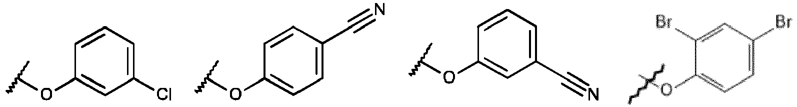 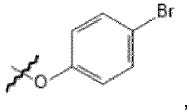 -CH₃, -CH₂CH₃, -CH₂CH₂CH₃, -C(CH₃)₃, -CF₃ and -CH₂CF₃.

Preferably, the compound of formula (I) is a compound selected from one of E.01 to E.20 in Table E (below).

In selected embodiments of the present invention, the compound of formula (I) is represented as, wherein where appropriate n is selected from 1, 2, 3 or 4:

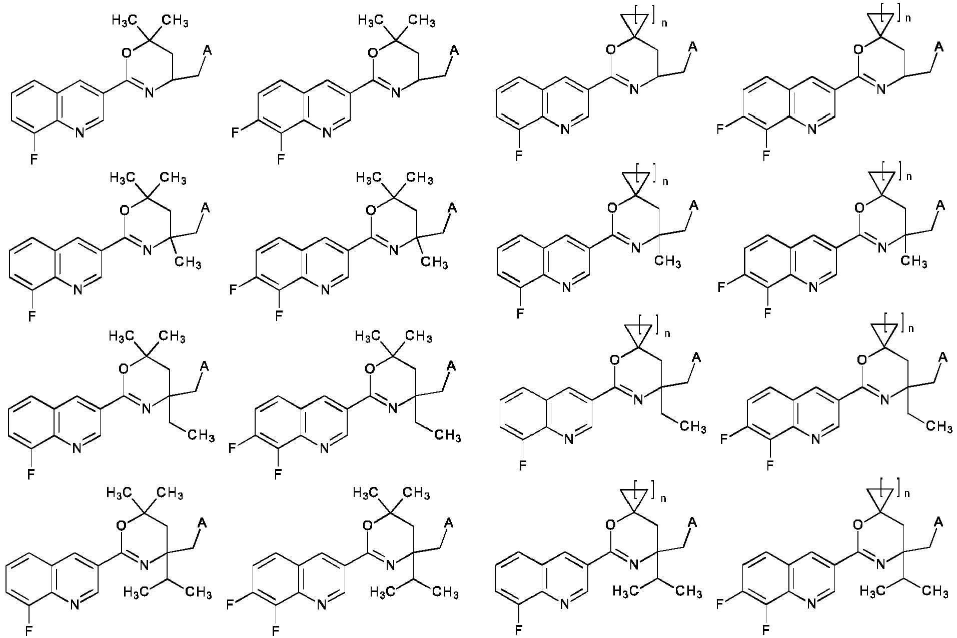

Specific examples of compounds of formula (I) are illustrated in the Tables A1 to A23, B1 to B23, C1 to C23 and D1 to D23 below:
Table A1: This table discloses 40 compounds of formula (I): 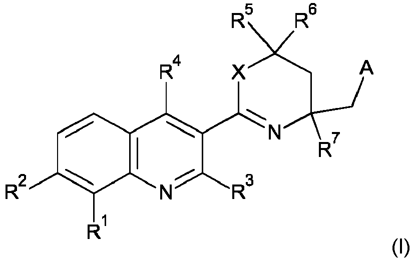 wherein R⁵ and R⁶ are -CH₃, R¹, R², R³, R⁴, X and R⁷ are as defined in Table Z below and A is:

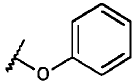

**Table Z**

| **Entry** | **R¹** | **R²** | **R³** | **R⁴** | **X** | **R⁷** |
|---|---|---|---|---|---|---|
| 1 | F | H | H | H | O | -CH₃ |
| 2 | F | F | H | H | O | -CH₃ |
| 3 | F | H | -CH₃ | H | O | -CH₃ |
| 4 | F | F | -CH₃ | H | O | -CH₃ |
| 5 | F | H | H | -CH₃ | O | -CH₃ |
| 6 | F | F | H | -CH₃ | O | -CH₃ |
| 7 | Cl | H | H | H | O | -CH₃ |
| 8 | Cl | F | H | H | O | -CH₃ |
| 9 | F | H | H | H | O | H |
| 10 | F | F | H | H | O | H |
| 11 | F | H | -CH₃ | H | O | H |
| 12 | F | F | -CH₃ | H | O | H |
| 13 | F | H | H | -CH₃ | O | H |
| 14 | F | F | H | -CH₃ | O | H |
| 15 | Cl | H | H | H | O | H |
| 16 | Cl | F | H | H | O | H |
| 17 | F | H | H | H | O | -CH₂CH₃ |
| 18 | F | F | H | H | O | -CH₂CH₃ |
| 19 | F | H | H | H | O | -CH(CH₃)₂ |
| 20 | F | F | H | H | O | -CH(CH₃)₂ |
| 21 | F | H | H | H | S | -CH₃ |
| 22 | F | F | H | H | S | -CH₃ |
| 23 | F | H | -CH₃ | H | S | -CH₃ |
| 24 | F | F | -CH₃ | H | S | -CH₃ |
| 25 | F | H | H | -CH₃ | s | -CH₃ |
| 26 | F | F | H | -CH₃ | s | -CH₃ |
| 27 | Cl | H | H | H | S | -CH₃ |
| 28 | Cl | F | H | H | S | -CH₃ |
| 29 | F | H | H | H | S | H |
| 30 | F | F | H | H | S | H |
| 31 | F | H | -CH₃ | H | S | H |
| 32 | F | F | -CH₃ | H | S | H |
| 33 | F | H | H | -CH₃ | S | H |
| 34 | F | F | H | -CH₃ | S | H |
| 35 | Cl | H | H | H | S | H |
| 36 | Cl | F | H | H | S | H |
| 37 | F | H | H | H | S | -CH₂CH₃ |
| 38 | F | F | H | H | S | -CH₂CH₃ |
| 39 | F | H | H | H | S | -CH(CH₃)₂ |
| 40 | F | F | H | H | S | -CH(CH₃)₂ |

Table A2 provides 40 compounds of formula (I) wherein R⁵ and R⁶ are -CH₃, R¹, R², R³, R⁴, X and R⁷ are as defined in Table Z above and wherein A is:

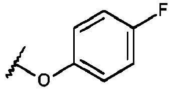

Table A3 provides 40 compounds of formula (I) wherein R⁵ and R⁶ are -CH₃, R¹, R², R³, R⁴, X and R⁷ are as defined in Table Z above and wherein A is:

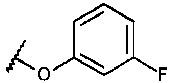

Table A4 provides 40 compounds of formula (I) wherein R⁵ and R⁶ are -CH₃, R¹, R², R³, R⁴, X and R⁷ are as defined in Table Z above and wherein A is:

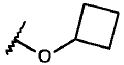

Table A5 provides 40 compounds of formula (I) wherein R⁵ and R⁶ are -CH₃, R¹, R², R³, R⁴, X and R⁷ are as defined in Table Z above and wherein A is:

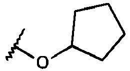

Table A6 provides 40 compounds of formula (I) wherein R⁵ and R⁶ are -CH₃, R¹, R², R³, R⁴, X and R⁷ are as defined in Table Z above and wherein A is:

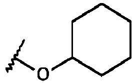

Table A7 provides 40 compounds of formula (I) wherein R⁵ and R⁶ are -CH₃, R¹, R², R³, R⁴, X and R⁷ are as defined in Table Z above and wherein A is:

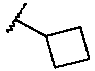

Table A8 provides 40 compounds of formula (I) wherein R⁵ and R⁶ are -CH₃, R¹, R², R³, R⁴, X and R⁷ are as defined in Table Z above and wherein A is:

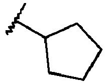

Table A9 provides 40 compounds of formula (I) wherein R⁵ and R⁶ are -CH₃, R¹, R², R³, R⁴, X and R⁷ are as defined in Table Z above and wherein A is:

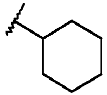

Table A10 provides 40 compounds of formula (I) wherein R⁵ and R⁶ are -CH₃, R¹, R², R³, R⁴, X and R⁷ are as defined in Table Z above and wherein A is:

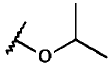

Table A11 provides 40 compounds of formula (I) wherein R⁵ and R⁶ are -CH₃, R¹, R², R³, R⁴, X and R⁷ are as defined in Table Z above and wherein A is:

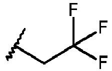

Table A12 provides 40 compounds of formula (I) wherein R⁵ and R⁶ are -CH₃, R¹, R², R³, R⁴, X and R⁷ are as defined in Table Z above and wherein A is:

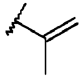

Table A13 provides 40 compounds of formula (I) wherein R⁵ and R⁶ are -CH₃, R¹, R², R³, R⁴, X and R⁷ are as defined in Table Z above and wherein A is:

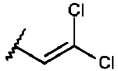

Table A14 provides 40 compounds of formula (I) wherein R⁵ and R⁶ are -CH₃, R¹, R², R³, R⁴, X and R⁷ are as defined in Table Z above and wherein A is:

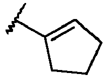

Table A15 provides 40 compounds of formula (I) wherein R⁵ and R⁶ are -CH₃, R¹, R², R³, R⁴, X and R⁷ are as defined in Table Z above and wherein A is:

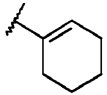

Table A16 provides 40 compounds of formula (I) wherein R⁵ and R⁶ are -CH₃, R¹, R², R³, R⁴, X and R⁷ are as defined in Table Z above and wherein A is:

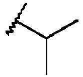

Table A17 provides 40 compounds of formula (I) wherein R⁵ and R⁶ are -CH₃, R¹, R², R³, R⁴, X and R⁷ are as defined in Table Z above and wherein A is:

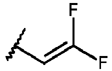

Table A18 provides 40 compounds of formula (I) wherein R⁵ and R⁶ are -CH₃, R¹, R², R³, R⁴, X and R⁷ are as defined in Table Z above and wherein A is:

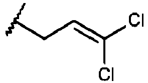

Table A19 provides 40 compounds of formula (I) wherein R⁵ and R⁶ are -CH₃, R¹, R², R³, R⁴, X and R⁷ are as defined in Table Z above and wherein A is:

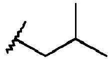

Table A20 provides 40 compounds of formula (I) wherein R⁵ and R⁶ are -CH₃, R¹, R², R³, R⁴, X and R⁷ are as defined in Table Z above and wherein A is:

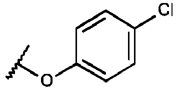

Table A21 provides 40 compounds of formula (I) wherein R⁵ and R⁶ are -CH₃, R¹, R², R³, R⁴, X and R⁷ are as defined in Table Z above and wherein A is:

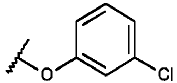

Table A22 provides 40 compounds of formula (I) wherein R⁵ and R⁶ are -CH₃, R¹, R², R³, R⁴, X and R⁷ are as defined in Table Z above and wherein A is:

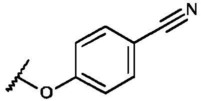

Table A23 provides 40 compounds of formula (I) wherein R⁵ and R⁶ are -CH₃, R¹, R², R³, R⁴, X and R⁷ are as defined in Table Z above and wherein A is:

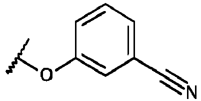

Tables B1 to B23 are constructed in an identical way as Tables A1 to A23. Each Table B1 to B23 provides 40 compounds of formula (I) wherein R⁵ is -CH₃ and R⁶ is -CH₂CH₃, R¹, R², R³, R⁴, X and R⁷ are as defined in Table Z above and wherein A is as defined for Tables A1 to A23.

Tables C1 to C23 are constructed in an identical way as Tables A1 to A23. Each Table C1 to C23 is providing 40 compounds of formula (I) wherein R⁵ and R⁶ together with their connecting carbon atom form a cyclobutane ring, R¹, R², R³, R⁴, X and R⁷ are as defined in Table Z above and wherein A is as defined for Tables A1 to A23.

Tables D1 to D23 are constructed in an identical way as Tables A1 to A23. Each Table D1 to D23 is providing 40 compounds of formula (I) wherein R⁵ and R⁶ together with their connecting carbon atom form a cyclopentane ring, R¹, R², R³, R⁴, X and R⁷ are as defined in Table Z above and wherein A is as defined for Tables A1 to A23.

Compounds of the present invention can be made as shown in the following schemes 1 to 11, in which, unless otherwise stated, the definition of each variable is as defined above for a compound of formula (I).

A shown in Scheme 1, compounds of general formula (I) can be prepared by treatment of compounds of general formula (II-b), with a protic acid such as trifluoroacetic acid, sulfuric acid, trifluoromethane sulfonic acid in an inert organic solvent such as acetonitrile, at temperatures between 20 °C and 80 °C.

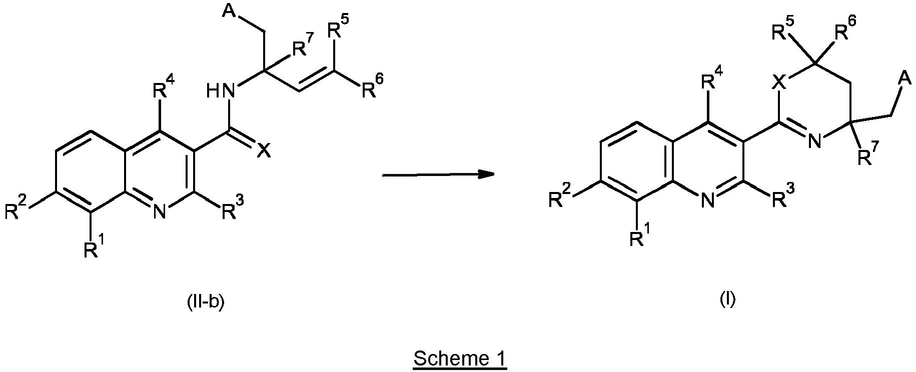

Alternatively, compounds of general formula (I) can be prepared by treatment of carboxamide compounds of formula (II-a) wherein R⁹ and R¹⁰ are hydrogen or C₁-C₄alkyl, or wherein R⁹ and R¹⁰ together with carbon atom to which they are attached form a C₃-C₅ cycloalkyl, or wherein R¹⁰ and R⁵ together with the two connecting carbon atoms form a cyclobutene or cyclopentene ring, with a protic acid such as trifluoroacetic acid, sulfuric acid, trifluoromethane sulfonic acid or halogenating agent such as *N*-chlorosuccinimide or select-fluor, in an organic solvent such as acetonitrile at temperatures between 20 °C and 80 °C, as described in Journal of Chemical Research, Synopses, (6), 200-1; 1986. This is shown in scheme 2.

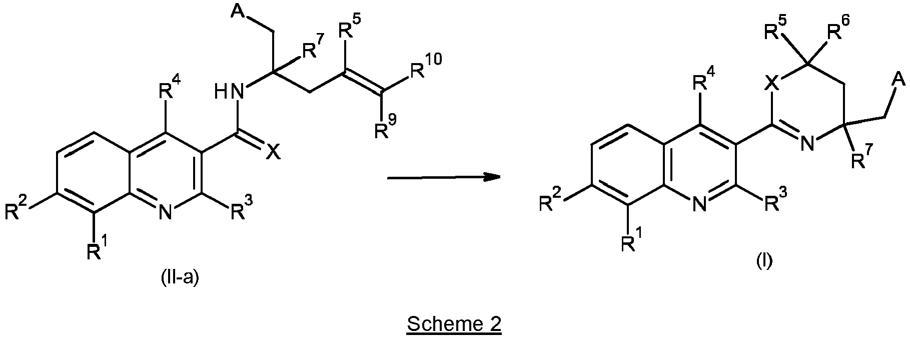

Compounds of general formula (II-a) and (II-b) wherein X is sulfur can be prepared from amides of general formula (II-c) and (II-d) respectively by treatment with a deoxothionating agent like P₄S₁₀ or Lawesson's reagent in an inert organic solvent like toluene at temperatures between 20 °C and 150 °C. This is shown in scheme 3.

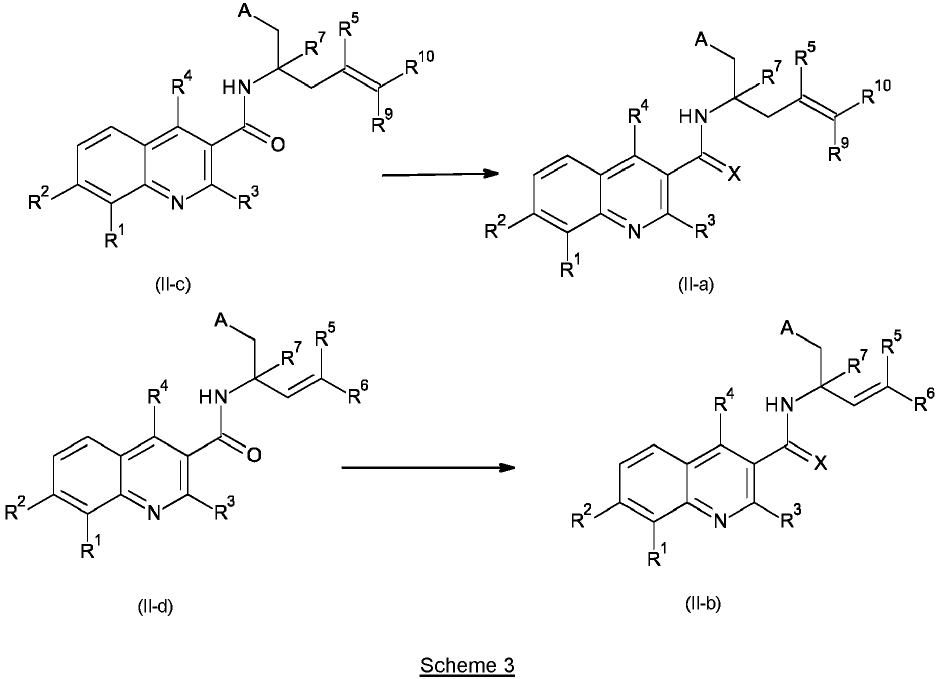

Compounds of general formula (II-c) and (II-d), wherein R⁹ and R¹⁰ are hydrogen or C₁-C₄alkyl or R⁹ and R¹⁰ together with the carbon atom to which they are attached form a C₃-C₅cycloalkyl, or wherein R¹⁰ and R⁵ together with the two connecting carbon atoms form a cyclobutene or cyclopentene ring, can be prepared from carboxylic acids of formula (III-a) and amines of formula (IV-a) or (IV-b) in the presence of an activating agent like phosgene or an amide coupling reagent like dicyclohexylcarbodiimide, in the presence or absence of a catalyst like N,N-dimethyl-4-aminopyridine in an inert organic solvent like tetrahydrofuran (THF). Such transformations are described in Chem. Soc. Rev., 2009, 606-631 and shown in scheme 4.

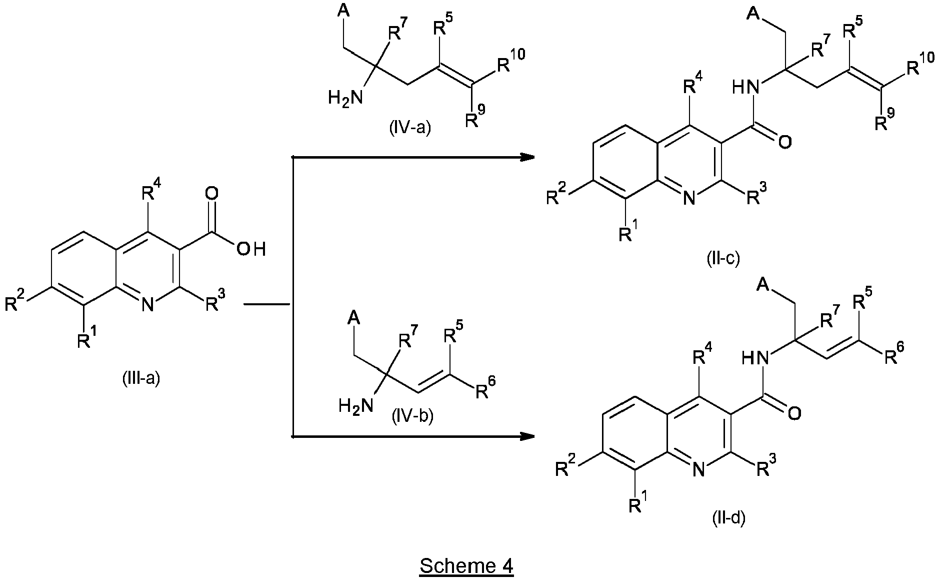

Alternatively, compounds of general formula (II-c) and (II-d) can be prepared by the reaction of quinoline (pseudo)halides of formula (III-b), wherein Y is Cl, Br, I or -OSO₂CF₃ with amines of formula (IV-a) or (IV-b) respectively, in the presence of carbon monoxide, a base like triethylamine or potassium carbonate, a transition metal (salt) such as palladium acetate and a suitable supporting ligand such as 4,5-Bis(diphenylphosphino)-9,9-dimethylxanthene in an inert organic solvent like 1,4-dioxane at a temperature between 20 °C and 110 °C as described in Org. Lett., 2014, 4296-4299 and references therein. This is shown in scheme 5.

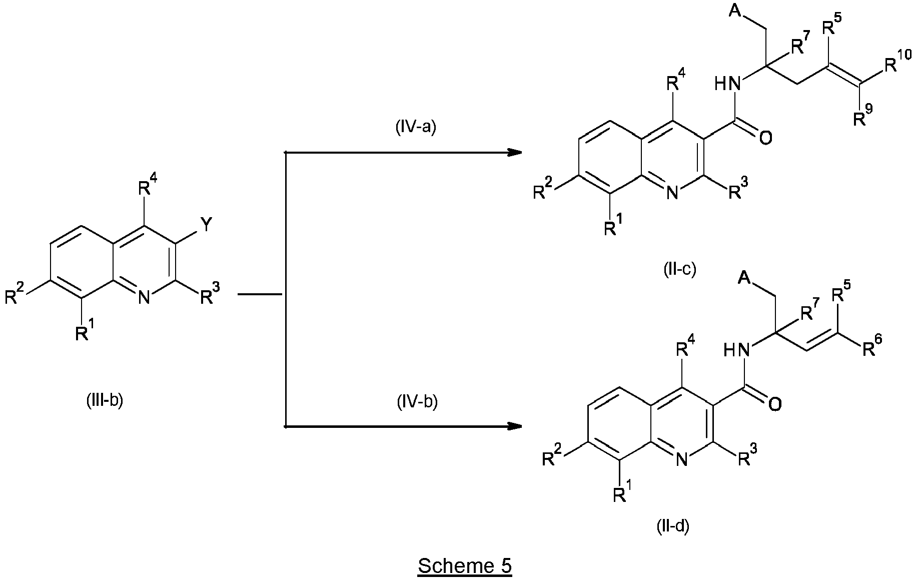

Alternatively, compounds of general formula (II-c) can also be prepared by the reaction of organometallic reagents of formula (III-c) wherein M is the salt of a metal selected from Mg, Zn, In, with isocyanates of formula (IV-c), wherein R⁹ and R¹⁰ are hydrogen or C₁-C₄alkyl, or wherein R⁹ and R¹⁰ together with carbon atom to which they are attached form a C₃-C₅cycloalkyl, or wherein R¹⁰ and R⁵ together with the two connecting carbon atoms form a cyclobutene or cyclopentene ring , in an inert organic solvent like THF at a temperature between 0 °C and 20 °C as described in Angew. Chem. Int. Ed. 2012, 51, 9173 - 9175 and references therein. This is shown in scheme 6.

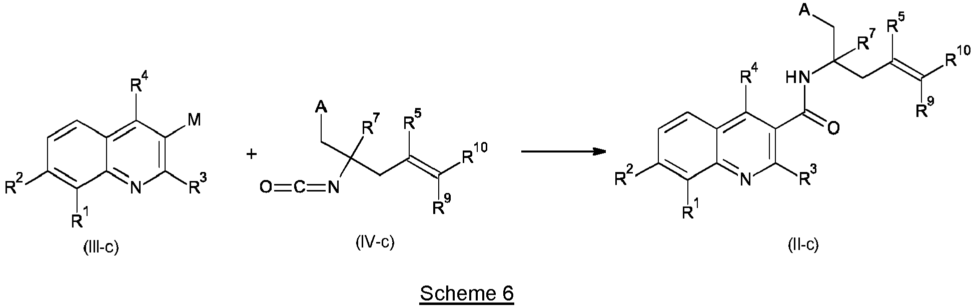

Compounds of general formula (IV-c) as defined above can be prepared from amines of formula (IV-a) as defined above, with a reagent like triphosgene and a base like triethylamine in an organic solvent like dichloromethane at a temperature between 0 °C and 20 °C as described in Synthetic Communications, 37: 1037-1044, 2007 and shown in scheme 7.

Alternatively, compounds of general formula (IV-c), can also be prepared via Lossen, Schmidt, Hoffmann or Curtius rearrangement of carboxylic acid derivates of formula (V-a), (V-b) or (V-c) respectively, wherein R⁹ and R¹⁰ are hydrogen or C₁-C₄alkyl, or wherein R⁹ and R¹⁰ together with carbon atom to which they are attached form a C₃-C₅cycloalkyl, or wherein R¹⁰ and R⁵ together with the two connecting carbon atoms form a cyclobutene or cyclopentene ring. These transformations to isocyanate (IV-c) and the conversion of carboxylic acids of formula (V-c) to derivatives of formula (V-a) and (V-b) can be performed according to standard protocols well known to a person skilled in the art, described in Synthesis, 2010, 2990-2996, J. Org. Chem. 2003, 68, 9453-9455, Chemistry Letters 2013, 42, 580-582, Synthesis, 2007, 3201-3204, Synlett, 2008, 2072-2074 and shown in scheme 7.

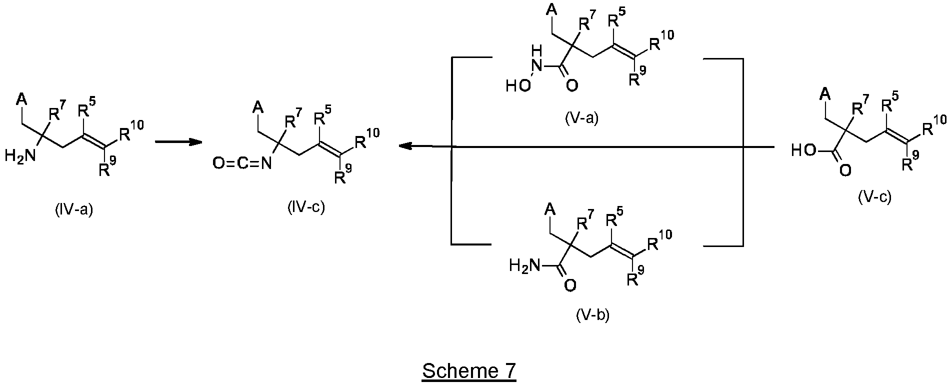

Alternatively, additional protocols for the preparation of aliphatic amines with formula (IV-a) as defined above have been reviewed in Chem. Rev. 2020, 120, 5, 2613-2692, *S*ynthesis 2017, 49, 776-789 and Chem. Rev. 2010, 110, 3600-3740 and references therein.

As shown in Scheme 8, compounds of general formula (VI-a), wherein R⁹ and R¹⁰ are hydrogen or C₁-C₄alkyl, or wherein R⁹ and R¹⁰ together with carbon atom to which they are attached form a C₃-C₅ cycloalkyl, or wherein R¹⁰ and R⁵ together with the two connecting carbon atoms form a cyclobutene or cyclopentene ring, and wherein R²⁰ is C₁-C₆alkyl or benzyl, can be prepared by functionalization of the *alpha* position of esters of formula (VII-a), wherein R²⁰ is as defined above, via deprotonation with a strong base like lithium diisopropylamide in an inert solvent like THF at temperatures between -78 °C and 20 °C followed by reaction with an electrophilic compound of formula (VII-b), wherein R⁹ and R¹⁰ together with carbon atom to which they are attached form a C₃-C₅cycloalkyl, or wherein R⁹ and R¹⁰ together with the two connecting carbon atoms form a cyclobutene or cyclopentene ring, and wherein Y is selected from C!, Br, I or OTf, to give an intermediate of formula (VII-c), wherein R⁹ and R¹⁰ together with carbon atom to which they are attached form a C₃-C₅cycloalkyl, or wherein R¹⁰ and R⁵ together with the two connecting carbon atoms form a cyclobutene or cyclopentene ring. This intermediate ester is then again deprotonated with a strong base like lithium diisopropylamine in an inert solvent like THF at temperatures between -78 °C and 20 °C followed by reaction with an electrophilic reagent like compounds of formula (VII-d) where A is as defined for compounds of formula (I) and wherein Y is selected from Cl, Br, I or OTf.

Alternatively, the same compounds of general formula (VI-a) as defined above, can be prepared by reversing the order of addition of the electrophilic reagents, via an intermediate ester of the formula (VII-e), wherein R²⁰ is C₁-C₆alkyl or benzyl.

Esters of general formula (VI-a) as defined above can be converted to carboxylic acids of formula (V-c) as defined above by methods well known to a person skilled in the art.

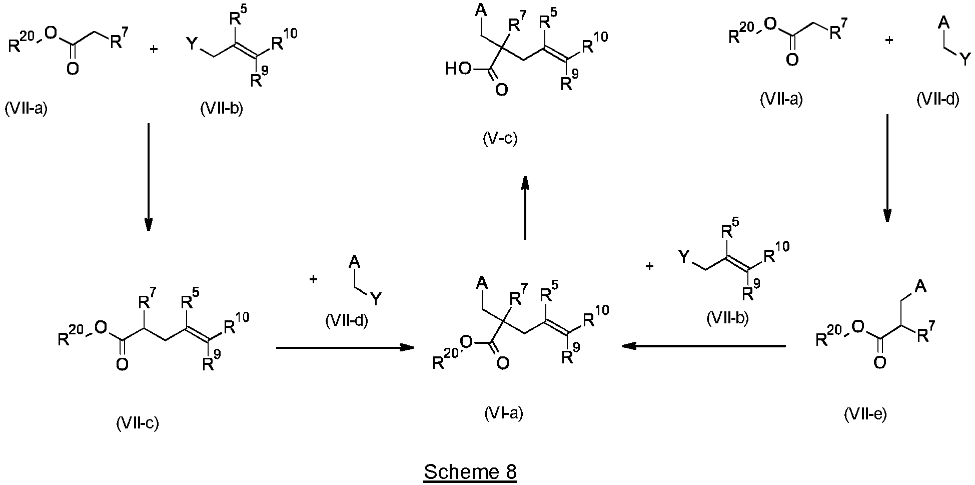

Primary amines of general formula (IV-b) can be obtained from sulfinamides of formula (V-d), wherein R¹¹ is C₁-C₆alkyl or an optionally substituted aryl, by treatment with a protic acid such as HCl in an organic solvent such as 1,4-dioxane or methanol as described in Tetrahedron Lett. 2009, 3482-3484. This is shown in scheme 9.

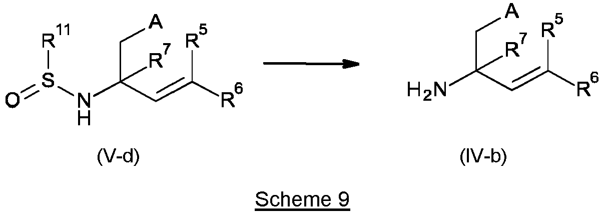

Sulfinamides of formula (V-d) as defined above can be prepared by nucleophilic addition of compounds of formula (VI-b), wherein M is the salt of a metal selected from Li, Mg, Zn, In, to sulfinyl imines of the formula (VI-c), wherein R¹¹ is C₁-C₆alkyl or an optionally substituted aryl, in an inert organic solvent like tetrahydrofuran. Alternatively, sulfinamides of formula (V-d) as defined above can be prepared by nucleophilic addition of compounds of formula (VI-e), wherein M is the salt of a metal selected from Li, Mg, Zn, In, to sulfinyl imines of the formula (Vl-f), wherein R¹¹ is C₁-C₆alkyl or an optionally substituted aryl, in an inert organic solvent like tetrahydrofuran. This is shown in scheme 10.

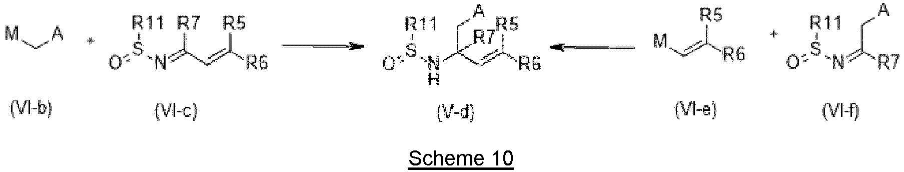

The general preparation of nucleophilic reagents with formula (VI-b) and (VI-e) as defined above is described in the scientific literature, synthetic chemistry textbooks such as March's Advanced Organic Chemistry, Smith and March, 6th edition, Wiley, 2007 and is generally known to a person skilled in the art. Furthermore, the methods for the preparation of sulfinyl imines of formula (VI-c) or (Vl-f) have been reviewed extensively in scientific publications such as Chem. Rev. 2010, 110, 3600-3740 and references therein, and are well known to a person skilled in the art.

Alternatively, amines of general formula (IV-b) can be prepared from of nitriles with general formula (VI-g) by sequential treatment with organometallic reagents of formula (VI-b) and (VI-e), wherein M is the salt of a metal selected from Li or Mg, in the presence or absence of a titanium salt such titanium isopropoxide in an inert solvent such THF. Alternatively, amines of general formula (IV-a) can be prepared from of nitriles with general formula (VI-g) by sequential treatment with organometallic reagents of formula (VI-h) and (VI-b), wherein M is the salt of a metal selected from Li or Mg, in the presence or absence of a titanium salt such titanium isopropoxide in an inert solvent such THF. This is described in Adv. Synth. Catal. 2017, 179 - 201 and shown in scheme 11.

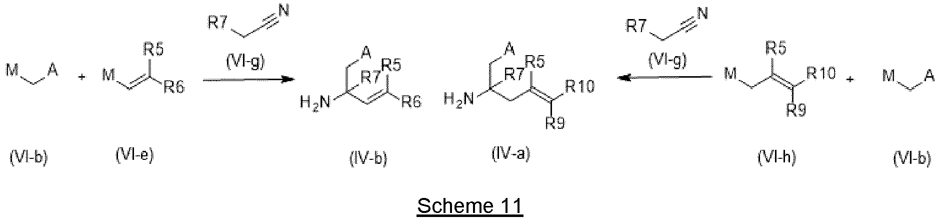

Alternatively, compounds of formula (I) can be obtained by transformation of another, closely related, compound of formula (I) (or analogue thereof) using standard synthesis techniques known to the person skilled in the art. Non-exhaustive examples include oxidation reactions, oxygenation reactions, reduction reactions, hydrogenation reactions, hydrolysis reactions, coupling reactions, aromatic nucleophilic or electrophilic substitution reactions, nucleophilic substitution reactions, deoxyfluorination reactions, alkylation reactions, radical additions, nucleophilic addition reactions, condensation and halogenation reactions.

Certain intermediates described in the above schemes are novel and as such form a further aspect of the invention.

The compounds of formula (I) can be used in the agricultural sector and related fields of use e.g. as active ingredients for controlling plant pests or on non-living materials for control of spoilage microorganisms or organisms potentially harmful to man. The novel compounds are distinguished by excellent activity at low rates of application, by being well tolerated by plants and by being environmentally safe. They have very useful curative, preventive and systemic properties and may be used for protecting numerous cultivated plants. The compounds of formula (I) can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula (I) as fungicide. The term "fungicide" as used herein means a compound that controls, modifies, or prevents the growth of fungi. The term "fungicidally effective amount" means the quantity of such a compound or combination of such compounds that is capable of producing an effect on the growth of fungi. Controlling or modifying effects include all deviation from natural development, such as killing, retardation and the like, and prevention includes barrier or other defensive formation in or on a plant to prevent fungal infection.

It is also possible to use compounds of formula (I) as dressing agents for the treatment of plant propagation material, *e*.*g*., seed, such as fruits, tubers or grains, or plant cuttings (for example rice), for the protection against fungal infections as well as against phytopathogenic fungi occurring in the soil. The propagation material can be treated with a composition comprising a compound of formula (I) before planting: seed, for example, can be dressed before being sown. The compounds of formula (I) can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore the compounds according to present invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

Compounds of formula (I) and fungicidal compositions containing them may be used to control plant diseases caused by a broad spectrum of fungal plant pathogens. They are effective in controlling a broad spectrum of plant diseases, such as foliar pathogens of ornamental, turf, vegetable, field, cereal, and fruit crops.

These fungi and fungal vectors of disease, as well as phytopathogenic bacteria and viruses, which may be controlled are for example:
Absidia corymbifera, Alternaria spp, Aphanomyces spp, Ascochyta spp, Aspergillus spp. including A. flavus, A. fumigatus, A. nidulans, A. niger, A. terrus, Aureobasidium spp. including A. pullulans, Blastomyces dermatitidis, Blumeria graminis, Bremia lactucae, Botryosphaeria spp. including B. dothidea, B. obtusa, Botrytis spp. inclusing B. cinerea, Candida spp. including C. albicans, C. glabrata, C. krusei, C. lusitaniae, C. parapsilosis, C. tropicalis, Cephaloascus fragrans, Ceratocystis spp, Cercospora spp. including C. arachidicola, Cercosporidium personatum, Cladosporium spp, Claviceps purpurea,
Coccidioides immitis, Cochliobolus spp, Colletotrichum spp. including C. musae,
Cryptococcus neoformans, Diaporthe spp, Didymella spp, Drechslera spp, Elsinoe spp,
Epidermophyton spp, Erwinia amylovora, Erysiphe spp. including E. cichoracearum,
Eutypa lata, Fusarium spp. including F. culmorum, F. graminearum, F. langsethiae, F. moniliforme, F. oxysporum, F. proliferatum, F. subglutinans, F. solani, Gaeumannomyces graminis, Gibberella fujikuroi, Gloeodes pomigena, Gloeosporium musarum, Glomerella cingulate, Guignardia bidwellii, Gymnosporangium juniperi-virginianae, Helminthosporium spp, Hemileia spp, Histoplasma spp. including H. capsulatum, Laetisaria fuciformis, Leptographium lindbergi, Leveillula taurica, Lophodermium seditiosum, Microdochium nivale, Microsporum spp, Monilinia spp, Mucor spp, Mycosphaerella spp. including M. graminicola, M. pomi, Oncobasidium theobromaeon, Ophiostoma piceae, Paracoccidioides spp, Penicillium spp. including P. digitatum, P. italicum, Petriellidium spp, Peronosclerospora spp. Including P. maydis, P. philippinensis and P. sorghi, Peronospora spp, Phaeosphaeria nodorum, Phakopsora pachyrhizi, Phellinus igniarus, Phialophora spp, Phoma spp, Phomopsis viticola, Phytophthora spp. including P. infestans, Plasmopara spp. including P. halstedii, P. viticola, Pleospora spp., Podosphaera spp. including P. leucotricha, Polymyxa graminis, Polymyxa betae, Pseudocercosporella herpotrichoides, Pseudomonas spp, Pseudoperonospora spp. including P. cubensis, P. humuli, Pseudopeziza tracheiphila, Puccinia Spp. including P. hordei, P. recondita, P. striiformis, P. triticina, Pyrenopeziza spp, Pyrenophora spp, Pyricularia spp. including P. oryzae, Pythium spp. including P. ultimum, Ramularia spp, Rhizoctonia spp, Rhizomucor pusillus, Rhizopus arrhizus, Rhynchosporium spp, Scedosporium spp. including S. apiospermum and S. prolificans, Schizothyrium pomi,
Sclerotinia spp, Sclerotium spp, Septoria spp, including S. nodorum, S. tritici, Sphaerotheca macularis, Sphaerotheca fusca (Sphaerotheca fuliginea), Sporothorix spp, Stagonospora nodorum, Stemphylium spp,. Stereum hirsutum, Thanatephorus cucumeris, Thielaviopsis basicola, Tilletia spp, Trichoderma spp. including T. harzianum, T. pseudokoningii, T. viride,

Trichophyton spp, Typhula spp, Uncinula necator, Urocystis spp, Ustilago spp, Venturia spp. including V. inaequalis, Verticillium spp, and Xanthomonas spp.

In particular, compounds of formula (I) and fungicidal compositions containing them may be used to control plant diseases caused by a broad spectrum of fungal plant pathogens in the Basidiomycete, Ascomycete, Oomycete and/or Deuteromycete, Blasocladiomycete, Chrytidiomycete, Glomeromycete and/or Mucoromycete classes.

These pathogens may include:
Oomycetes, including Phytophthora diseases such as those caused by *Phytophthora capsici, Phytophthora infestans, Phytophthora sojae, Phytophthora fragariae*, *Phytophthora nicotianae, Phytophthora cinnamomi, Phytophthora citricola, Phytophthora citrophthora* and *Phytophthora erythroseptica;* Pythium diseases such as those caused by *Pythium aphanidermatum*, *Pythium arrhenomanes, Pythium graminicola, Pythium irregulare* and *Pythium ultimum;* diseases caused by Peronosporales such as *Peronospora destructor, Peronospora parasitica, Plasmopara viticola, Plasmopara halstedii, Pseudoperonospora cubensis, Albugo candida, Sclerophthora macrospora* and *Bremia lactucae;* and others such as *Aphanomyces cochlioides, Labyrinthula zosterae, Peronosclerospora sorghi* and *Sclerospora graminicola.*
Ascomycetes, including blotch, spot, blast or blight diseases and/or rots for example those caused by Pleosporales such as *Stemphylium solani, Stagonospora tainanensis, Spilocaea oleaginea, Setosphaeria turcica, Pyrenochaeta lycoperisici, Pleospora herbarum, Phoma destructiva, Phaeosphaeria herpotrichoides, Phaeocryptocus gaeumannii, Ophiosphaerella graminicola, Ophiobolus graminis, Leptosphaeria maculans, Hendersonia creberrima, Helminthosporium triticirepentis*, *Setosphaeria turcica, Drechslera glycines, Didymella bryoniae, Cycloconium oleagineum, Corynespora cassiicola, Cochliobolus sativus, Bipolaris cactivora, Venturia inaequalis, Pyrenophora teres, Pyrenophora tritici-repentis*, *Alternaria alternata, Alternaria brassicicola, Alternaria solani* and *Alternaria tomatophila,* Capnodiales such as *Septoria tritici, Septoria nodorum, Septoria glycines, Cercospora arachidicola, Cercospora sojina, Cercospora zeae-maydis, Cercosporella capsellae* and *Cercosporella herpotrichoides, Cladosporium carpophilum, Cladosporium effusum, Passalora fulva, Cladosporium oxysporum, Dothistroma septosporum, Isariopsis clavispora, Mycosphaerella fijiensis, Mycosphaerella graminicola, Mycovellosiella koepkeii, Phaeoisariopsis bataticola, Pseudocercospora vitis, Pseudocercosporella herpotrichoides, Ramularia beticola, Ramularia collo-cygni,* Magnaporthales such as *Gaeumannomyces graminis, Magnaporthe grisea, Pyricularia oryzae,* Diaporthales such as *Anisogramma anomala, Apiognomonia errabunda*, *Cytospora platani, Diaporthe phaseolorum, Discula destructiva, Gnomonia fructicola, Greeneria uvicola, Melanconium juglandinum, Phomopsis viticola, Sirococcus clavigignenti-juglandacearum, Tubakia dryina, Dicarpella* spp., *Valsa ceratosperma*, and others such as *Actinothyrium graminis, Ascochyta pisi, Aspergillus flavus, Aspergillus fumigatus, Aspergillus nidulans, Asperisporium caricae*, *Blumeriella jaapii, Candida* spp., *Capnodium ramosum, Cephaloascus* spp., *Cephalosporium gramineum, Ceratocystis paradoxa, Chaetomium* spp., *Hymenoscyphus pseudoalbidus, Coccidioides* spp., *Cylindrosporium padi, Diplocarpon malae, Drepanopeziza campestris, Elsinoe ampelina, Epicoccum nigrum, Epidermophyton* spp., *Eutypa lata, Geotrichum candidum, Gibellina cerealis, Gloeocercospora sorghi, Gloeodes pomigena, Gloeosporium perennans; Gloeotinia temulenta, Griphospaeria corticola, Kabatiella lini, Leptographium microsporum, Leptosphaerulinia crassiasca, Lophodermium seditiosum, Marssonina graminicola, Microdochium nivale, Monilinia fructicola, Monographella albescens, Monosporascus cannonballus, Naemacyclus* spp., *Ophiostoma novo-ulmi, Paracoccidioides brasiliensis, Penicillium expansum, Pestalotia rhododendri, Petriellidium* spp., *Pezicula* spp., *Phialophora gregata, Phyllachora pomigena, Phymatotrichum omnivora, Physalospora abdita, Plectosporium tabacinum, Polyscytalum pustulans, Pseudopeziza medicaginis, Pyrenopeziza brassicae, Ramulispora sorghi, Rhabdocline pseudotsugae, Rhynchosporium secalis, Sacrocladium oryzae, Scedosporium* spp., *Schizothyrium pomi, Sclerotinia sclerotiorum, Sclerotinia minor; Sclerotium* spp., *Typhula ishikariensis, Seimatosporium mariae, Lepteutypa cupressi, Septocyta ruborum, Sphaceloma perseae, Sporonema phacidioides, Stigmina palmivora, Tapesia yallundae, Taphrina bullata, Thielviopsis basicola, Trichoseptoria fructigena*, *Zygophiala jamaicensis;* powdery mildew diseases for example those caused by Erysiphales such as *Blumeria graminis, Erysiphe polygoni, Uncinula necator, Sphaerotheca fuligena, Podosphaera leucotricha*, *Podospaera macularis Golovinomyces cichoracearum, Leveillula taurica, Microsphaera diffusa, Oidiopsis gossypii, Phyllactinia guttata* and *Oidium arachidis;* molds for example those caused by Botryosphaeriales such as *Dothiorella aromatica, Diplodia seriata, Guignardia bidwellii, Botrytis cinerea, Botryotinia allii*, *Botryotinia fabae, Fusicoccum amygdali, Lasiodiplodia theobromae, Macrophoma theicola, Macrophomina phaseolina, Phyllosticta cucurbitacearum;* anthracnoses for example those caused by Glommerelales such as *Colletotrichum gloeosporioides, Colletotrichum lagenarium, Colletotrichum gossypii, Glomerella cingulata,* and *Colletotrichum graminicola;* and wilts or blights for example those caused by Hypocreales such as *Acremonium strictum, Claviceps purpurea, Fusarium culmorum, Fusarium graminearum, Fusarium virguliforme, Fusarium oxysporum, Fusarium subglutinans, Fusarium oxysporum* f.sp. *cubense, Gerlachia nivale, Gibberella fujikuroi, Gibberella zeae, Gliocladium* spp., *Myrothecium verrucaria, Nectria ramulariae, Trichoderma viride, Trichothecium roseum,* and *Verticillium theobromae.*
Basidiomycetes, including smuts for example those caused by Ustilaginales such as *Ustilaginoidea virens, Ustilago nuda, Ustilago tritici, Ustilago zeae,* rusts for example those caused by Pucciniales such as *Cerotelium fici, Chrysomyxa arctostaphyli, Coleosporium ipomoeae, Hemileia vastatrix, Puccinia arachidis, Puccinia cacabata, Puccinia graminis, Puccinia recondita, Puccinia sorghi, Puccinia hordei, Puccinia striiformis* f.sp. Hordei, *Puccinia striiformis* f.sp. Secalis, *Pucciniastrum coryli,* or Uredinales such as *Cronartium ribicola, Gymnosporangium juniperi-viginianae, Melampsora medusae, Phakopsora pachyrhizi, Phragmidium mucronatum, Physopella ampelosidis, Tranzschelia discolor* and *Uromyces viciae-fabae;* and other rots and diseases such as those caused by *Cryptococcus* spp., *Exobasidium vexans, Marasmiellus inoderma*, *Mycena* spp., *Sphacelotheca reiliana, Typhula ishikariensis, Urocystis agropyri, Itersonilia perplexans, Corticium invisum, Laetisaria fuciformis*, *Waitea circinata, Rhizoctonia solani, Thanetephorus cucurmeris, Entyloma dahliae, Entylomella microspora, Neovossia moliniae* and *Tilletia caries.*
Blastocladiomycetes, such as *Physoderma maydis.*
Mucoromycetes, such as *Choanephora cucurbitarum.; Mucorspp.; Rhizopus arrhizus,*

As well as diseases caused by other species and genera closely related to those listed above.

In addition to their fungicidal activity, the compounds and compositions comprising them may also have activity against bacteria such as *Erwinia amylovora, Erwinia caratovora, Xanthomonas campestris, Pseudomonas syringae, Strptomyces scabies* and other related species as well as certain protozoa.

Within the scope of present invention, target crops and/or useful plants to be protected typically comprise perennial and annual crops, such as berry plants for example blackberries, blueberries, cranberries, raspberries and strawberries; cereals for example barley, maize (corn), millet, oats, rice, rye, sorghum triticale and wheat; fibre plants for example cotton, flax, hemp, jute and sisal; field crops for example sugar and fodder beet, coffee, hops, mustard, oilseed rape (canola), poppy, sugar cane, sunflower, tea and tobacco; fruit trees for example apple, apricot, avocado, banana, cherry, citrus, nectarine, peach, pear and plum; grasses for example Bermuda grass, bluegrass, bentgrass, centipede grass, fescue, ryegrass, St. Augustine grass and Zoysia grass; herbs such as basil, borage, chives, coriander, lavender, lovage, mint, oregano, parsley, rosemary, sage and thyme; legumes for example beans, lentils, peas and soya beans; nuts for example almond, cashew, ground nut, hazelnut, peanut, pecan, pistachio and walnut; palms for example oil palm; ornamentals for example flowers, shrubs and trees; other trees, for example cacao, coconut, olive and rubber; vegetables for example asparagus, aubergine, broccoli, cabbage, carrot, cucumber, garlic, lettuce, marrow, melon, okra, onion, pepper, potato, pumpkin, rhubarb, spinach and tomato; and vines for example grapes.

The useful plants and / or target crops in accordance with the invention include conventional as well as genetically enhanced or engineered varieties such as, for example, insect resistant (e.g. Bt. and VIP varieties) as well as disease resistant, herbicide tolerant (e.g. glyphosate- and glufosinate-resistant maize varieties commercially available under the trade names RoundupReady^{®} and LibertyLink^{®}) and nematode tolerant varieties. By way of example, suitable genetically enhanced or engineered crop varieties include the Stoneville 5599BR cotton and Stoneville 4892BR cotton varieties.

The term "useful plants" and/or "target crops" is to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®} , Herculex I^{®} and LibertyLink^{®}.

The term "useful plants" and/or "target crops" is to be understood as including those which naturally are or have been rendered resistant to harmful insects. This includes plants transformed by the use of recombinant DNA techniques, for example, to be capable of synthesising one or more selectively acting toxins, such as are known, for example, from toxin-producing bacteria. Examples of toxins which can be expressed include δ-endotoxins, vegetative insecticidal proteins (Vip), insecticidal proteins of bacteria colonising nematodes, and toxins produced by scorpions, arachnids, wasps and fungi. An example of a crop that has been modified to express the *Bacillus thuringiensis* toxin is the Bt maize KnockOut^{®} (Syngenta Seeds). An example of a crop comprising more than one gene that codes for insecticidal resistance and thus expresses more than one toxin is VipCot^{®} (Syngenta Seeds). Crops or seed material thereof can also be resistant to multiple types of pests (so-called stacked transgenic events when created by genetic modification). For example, a plant can have the ability to express an insecticidal protein while at the same time being herbicide tolerant, for example Herculex I^{®} (Dow AgroSciences, Pioneer Hi-Bred International).

The term "useful plants" and/or "target crops" is to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

Toxins that can be expressed by transgenic plants include, for example, insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

Further, in the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO03/018810).

More examples of such toxins or transgenic plants capable of synthesising such toxins are disclosed, for example, in EP-A-0 374 753, WO93/07278, WO95/34656, EP-A-0 427 529, EP-A-451 878 and WO03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603** × **MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

Pesticidal agents referred to herein using their common name are known, for example, from "The Pesticide Manual", 15th Ed., British Crop Protection Council 2009.

The compounds of formula (I) may be used in unmodified form or, preferably, together with the adjuvants conventionally employed in the art of formulation. To this end they may be conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g. for agricultural use, can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

Suspension concentrates are aqueous formulations in which finely divided solid particles of the active compound are suspended. Such formulations include anti-settling agents and dispersing agents and may further include a wetting agent to enhance activity as well an anti-foam and a crystal growth inhibitor. In use, these concentrates are diluted in water and normally applied as a spray to the area to be treated. The amount of active ingredient may range from 0.5% to 95% of the concentrate.

Wettable powders are in the form of finely divided particles which disperse readily in water or other liquid carriers. The particles contain the active ingredient retained in a solid matrix. Typical solid matrices include fuller's earth, kaolin clays, silicas and other readily wet organic or inorganic solids. Wettable powders normally contain from 5% to 95% of the active ingredient plus a small amount of wetting, dispersing or emulsifying agent.

Emulsifiable concentrates are homogeneous liquid compositions dispersible in water or other liquid and may consist entirely of the active compound with a liquid or solid emulsifying agent, or may also contain a liquid carrier, such as xylene, heavy aromatic naphthas, isophorone and other non-volatile organic solvents. In use, these concentrates are dispersed in water or other liquid and normally applied as a spray to the area to be treated. The amount of active ingredient may range from 0.5% to 95% of the concentrate.

Granular formulations include both extrudates and relatively coarse particles and are usually applied without dilution to the area in which treatment is required. Typical carriers for granular formulations include sand, fuller's earth, attapulgite clay, bentonite clays, montmorillonite clay, vermiculite, perlite, calcium carbonate, brick, pumice, pyrophyllite, kaolin, dolomite, plaster, wood flour, ground corn cobs, ground peanut hulls, sugars, sodium chloride, sodium sulphate, sodium silicate, sodium borate, magnesia, mica, iron oxide, zinc oxide, titanium oxide, antimony oxide, cryolite, gypsum, diatomaceous earth, calcium sulphate and other organic or inorganic materials which absorb or which can be coated with the active compound. Granular formulations normally contain 5% to 25% of active ingredients which may include surface-active agents such as heavy aromatic naphthas, kerosene and other petroleum fractions, or vegetable oils; and/or stickers such as dextrins, glue or synthetic resins.

Dusts are free-flowing admixtures of the active ingredient with finely divided solids such as talc, clays, flours and other organic and inorganic solids which act as dispersants and carriers.

Microcapsules are typically droplets or granules of the active ingredient enclosed in an inert porous shell which allows escape of the enclosed material to the surroundings at controlled rates. Encapsulated droplets are typically 1 to 50 microns in diameter. The enclosed liquid typically constitutes 50 to 95% of the weight of the capsule and may include solvent in addition to the active compound. Encapsulated granules are generally porous granules with porous membranes sealing the granule pore openings, retaining the active species in liquid form inside the granule pores. Granules typically range from 1 millimetre to 1 centimetre and preferably 1 to 2 millimetres in diameter. Granules are formed by extrusion, agglomeration or prilling, or are naturally occurring. Examples of such materials are vermiculite, sintered clay, kaolin, attapulgite clay, sawdust and granular carbon. Shell or membrane materials include natural and synthetic rubbers, cellulosic materials, styrene-butadiene copolymers, polyacrylonitriles, polyacrylates, polyesters, polyamides, polyureas, polyurethanes and starch xanthates.

Other useful formulations for agrochemical applications include simple solutions of the active ingredient in a solvent in which it is completely soluble at the desired concentration, such as acetone, alkylated naphthalenes, xylene and other organic solvents. Pressurised sprayers, wherein the active ingredient is dispersed in finely-divided form as a result of vaporisation of a low boiling dispersant solvent carrier, may also be used.

Suitable agricultural adjuvants and carriers that are useful in formulating the compositions of the invention in the formulation types described above are well known to those skilled in the art.

Liquid carriers that can be employed include, for example, water, toluene, xylene, petroleum naphtha, crop oil, acetone, methyl ethyl ketone, cyclohexanone, acetic anhydride, acetonitrile, acetophenone, amyl acetate, 2-butanone, chlorobenzene, cyclohexane, cyclohexanol, alkyl acetates, diacetonalcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethyl formamide, dimethyl sulfoxide, 1,4-dioxane, dipropylene glycol, dipropylene glycol methyl ether, dipropylene glycol dibenzoate, diproxitol, alkyl pyrrolidinone, ethyl acetate, 2-ethyl hexanol, ethylene carbonate, 1,1,1-trichloroethane, 2-heptanone, alpha pinene, d-limonene, ethylene glycol, ethylene glycol butyl ether, ethylene glycol methyl ether, gamma-butyrolactone, glycerol, glycerol diacetate, glycerol monoacetate, glycerol triacetate, hexadecane, hexylene glycol, isoamyl acetate, isobornyl acetate, isooctane, isophorone, isopropyl benzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, m-xylene, n-hexane, n-octylamine, octadecanoic acid, octyl amine acetate, oleic acid, oleylamine, o-xylene, phenol, polyethylene glycol (PEG400), propionic acid, propylene glycol, propylene glycol monomethyl ether, p-xylene, toluene, triethyl phosphate, triethylene glycol, xylene sulfonic acid, paraffin, mineral oil, trichloroethylene, perchloroethylene, ethyl acetate, amyl acetate, butyl acetate, methanol, ethanol, isopropanol, and higher molecular weight alcohols such as amyl alcohol, tetrahydrofurfuryl alcohol, hexanol, octanol, etc., ethylene glycol, propylene glycol, glycerine and N-methyl-2-pyrrolidinone. Water is generally the carrier of choice for the dilution of concentrates.

Suitable solid carriers include, for example, talc, titanium dioxide, pyrophyllite clay, silica, attapulgite clay, kieselguhr, chalk, diatomaxeous earth, lime, calcium carbonate, bentonite clay, fuller's earth, cotton seed hulls, wheat flour, soybean flour, pumice, wood flour, walnut shell flour and lignin.

A broad range of surface-active agents are advantageously employed in both said liquid and solid compositions, especially those designed to be diluted with carrier before application. These agents, when used, normally comprise from 0.1% to 15% by weight of the formulation. They can be anionic, cationic, non-ionic or polymeric in character and can be employed as emulsifying agents, wetting agents, suspending agents or for other purposes. Typical surface active agents include salts of alkyl sulfates, such as diethanolammonium lauryl sulphate; alkylarylsulfonate salts, such as calcium dodecylbenzenesulfonate; alkylphenol-alkylene oxide addition products, such as nonylphenol-C.sub. 18 ethoxylate; alcohol-alkylene oxide addition products, such as tridecyl alcohol-C.sub. 16 ethoxylate; soaps, such as sodium stearate; alkylnaphthalenesulfonate salts, such as sodium dibutylnaphthalenesulfonate; dialkyl esters of sulfosuccinate salts, such as sodium di(2-ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; and salts of mono and dialkyl phosphate esters.

Other adjuvants commonly utilized in agricultural compositions include crystallisation inhibitors, viscosity modifiers, suspending agents, spray droplet modifiers, pigments, antioxidants, foaming agents, anti-foaming agents, light-blocking agents, compatibilizing agents, antifoam agents, sequestering agents, neutralising agents and buffers, corrosion inhibitors, dyes, odorants, spreading agents, penetration aids, micronutrients, emollients, lubricants and sticking agents.

In addition, further, other biocidally active ingredients or compositions may be combined with the compositions of the invention and used in the methods of the invention and applied simultaneously or sequentially with the compositions of the invention. When applied simultaneously, these further active ingredients may be formulated together with the compositions of the invention or mixed in, for example, the spray tank. These further biocidally active ingredients may be fungicides, herbicides, insecticides, bactericides, acaricides, nematicides and/or plant growth regulators.

In addition, the compositions of the invention may also be applied with one or more systemically acquired resistance inducers ("SAR" inducer). SAR inducers are known and described in, for example, United States Patent No. US 6,919,298 and include, for example, salicylates and the commercial SAR inducer acibenzolar-S-methyl.

The compounds of formula (I) are normally used in the form of compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula (I) may be used in the form of (fungicidal) compositions for controlling or protecting against phytopathogenic microorganisms, comprising as active ingredient at least one compound of formula (I) or of at least one preferred individual compound as above-defined, in free form or in agrochemically usable salt form, and at least one of the above-mentioned adjuvants.

The invention therefore provides a composition, preferably a fungicidal composition, comprising at least one compound formula (I) an agriculturally acceptable carrier and optionally an adjuvant. An agricultural acceptable carrier is for example a carrier that is suitable for agricultural use. Agricultural carriers are well known in the art. Preferably said composition may comprise at least one or more pesticidally active compounds, for example an additional fungicidal active ingredient in addition to the compound of formula (I).

The compound of formula (I) may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

Examples of suitable additional active ingredients include the following: 1,2,4-thiadiazoles, 2,6-dinitroanilines, acylalanines, aliphatic nitrogenous compounds, amidines, aminopyrimidinols, anilides, anilino-pyrimidines, anthraquinones, antibiotics, aryl-phenylketones, benzamides, benzene-sulfonamides, benzimidazoles, benzothiazoles, benzothiodiazoles, benzothiophenes, benzoylpyridines, benzthiadiazoles, benzylcarbamates, butylamines, carbamates, carboxamides, carpropamids, chloronitriles, cinnamic acid amides, copper containing compounds, cyanoacetamideoximes, cyanoacrylates, cyanoimidazoles, cyanomethylene-thiazolidines, dicarbonitriles, dicarboxamides, dicarboximides, dimethylsulphamates, dinitrophenol carbonates, dinitrophenysl, dinitrophenyl crotonates, diphenyl phosphates, dithiino compounds, dithiocarbamates, dithioethers, dithiolanes, ethyl-amino-thiazole carboxamides, ethyl-phosphonates, furan carboxamides, glucopyranosyls, glucopyranoxyls, glutaronitriles, guanidines, herbicides/plant growth regulatosr, hexopyranosyl antibiotics, hydroxy(2-amino)pyrimidines, hydroxyanilides, hydroxyisoxazoles, imidazoles, imidazolinones, insecticides/plant growth regulators, isobenzofuranones, isoxazolidinyl-pyridines, isoxazolines, maleimides, mandelic acid amides, mectin derivatives, morpholines, norpholines, n-phenyl carbamates, organotin compounds, oxathiin carboxamides, oxazoles, oxazolidine-diones, phenols, phenoxy quinolines, phenyl-acetamides, phenylamides, phenylbenzamides, phenyl-oxo-ethyl-thiophenes amides, phenylpyrroles, phenylureas, phosphorothiolates, phosphorus acids, phthalamic acids, phthalimides, picolinamides, piperazines, piperidines, plant extracts, polyoxins, propionamides, pthalimides, pyrazole-4-carboxamides, pyrazolinones, pyridazinones, pyridines, pyridine carboxamides, pyridinyl-ethyl benzamides, pyrimdinamines, pyrimidines, pyrimidine-amines, pyrimidione-hydrazone, pyrrolidines, pyrrolquinoliones, quinazolinones, quinolines, quinoline derivatives, quinoline-7-carboxylic acids, quinoxalines, spiroketalamines, strobilurins, sulfamoyl triazoles, sulphamides, tetrazolyloximes, thiadiazines, thiadiazole carboxamides, thiazole carboxanides, thiocyanates, thiophene carboxamides, toluamides, triazines, triazobenthiazoles, triazoles, triazole-thiones, triazolo-pyrimidylamine, valinamide carbamates, ammonium methyl phosphonates, arsenic-containing compounds, benyimidazolylcarbamates, carbonitriles, carboxanilides, carboximidamides, carboxylic phenylamides, diphenyl pyridines, furanilides, hydrazine carboxamides, imidazoline acetates, isophthalates, isoxazolones, mercury salts, organomercury compounds, organophosphates, oxazolidinediones, pentylsulfonyl benzenes, phenyl benzamides, phosphonothionates, phosphorothioates, pyridyl carboxamides, pyridyl furfuryl ethers, pyridyl methyl ethers, SDHls, thiadiazinanethiones, thiazolidines..

Examples of suitable additional active ingredients also include the following: a compound selected from the group of substances consisting of petroleum oils, 1,1-bis(4-chlorophenyl)-2-ethoxyethanol, 2,4-dichlorophenyl benzenesulfonate, 2-fluoro-N-methyl-N-1-naphthylacetamide, 4-chlorophenyl phenyl sulfone, acetoprole, aldoxycarb, amidithion, amidothioate, amiton, amiton hydrogen oxalate, amitraz, aramite, arsenous oxide, azobenzene, azothoate, benomyl, benoxafos, benzyl benzoate, bixafen, brofenvalerate, bromocyclen, bromophos, bromopropylate, buprofezin, butocarboxim, butoxycarboxim, butylpyridaben, calcium polysulfide, camphechlor, carbanolate, carbophenothion, cymiazole, chino-methionat, chlorbenside, chlordimeform, chlordimeform hydrochloride, chlorfenethol, chlorfenson, chlorfensulfide, chlorobenzilate, chloromebuform, chloromet hiuron, chloropropylate, chlorthiophos, cinerin I, cinerin II, cinerins, closantel, coumaphos, crotamiton, crotoxyphos, cufraneb, cyanthoate, DCPM, DDT, demephion, demephion-O, demephion-S, demeton-methyl, demeton-O, demeton-O-methyl, demeton-S, demeton-S-methyl, demeton-S-methylsulfon, dichlofluanid, dichlorvos, dicliphos, dienochlor, dimefox, dinex, dinex-diclexine, dinocap-4, dinocap-6, dinocton, dinopenton, dinosulfon, dinoterbon, dioxathion, diphenyl sulfone, disulfiram, DNOC, dofenapyn, doramectin, endothion, eprinomectin, ethoate-methyl, etrimfos, fenazaflor, fenbutatin oxide, fenothiocarb, fenpyrad, fenpyroximate, fenpyrazamine, fenson, fentrifanil, flubenzimine, flucycloxuron, fluenetil, fluorbenside, FMC 1137, formetanate, formetanate hydrochloride, formparanate, gamma-HCH, glyodin, halfenprox, hexadecyl cyclopropanecarboxylate, isocarbophos, jasmolin I, jasmolin II, jodfenphos, lindane, malonoben, mecarbam, mephosfolan, mesulfen, methacrifos, methyl bromide, metolcarb, mexacarbate, milbemycin oxime, mipafox, monocrotophos, morphothion, moxidectin, naled, 4-chloro-2-(2-chloro-2-methyl-propyl)-5-[(6-iodo-3-pyridyl)methoxy]pyridazin-3-one, nifluridide, nikkomycins, nitrilacarb, nitrilacarb 1:1 zinc chloride complex, omethoate, oxydeprofos, oxydisulfoton, pp'-DDT, parathion, permethrin, phenkapton, phosalone, phosfolan, phosphamidon, polychloroterpenes, polynactins, proclonol, promacyl, propoxur, prothidathion, prothoate, pyrethrin I, pyrethrin II, pyrethrins, pyridaphenthion, pyrimitate, quinalphos, quintiofos, R-1492, phosglycin, rotenone, schradan, sebufos, selamectin, sophamide, SSI-121, sulfiram, sulfluramid, sulfotep, sulfur, diflovidazin, tau-fluvalinate, TEPP, terbam, tetradifon, tetrasul, thiafenox, thiocarboxime, thiofanox, thiometon, thioquinox, thuringiensin, triamiphos, triarathene, triazophos, triazuron, trifenofos, trinactin, vamidothion, vaniliprole, bethoxazin, copper dioctanoate, copper sulfate, cybutryne, dichlone, dichlorophen, endothal, fentin, hydrated lime, nabam, quinoclamine, quinonamid, simazine, triphenyltin acetate, triphenyltin hydroxide, crufomate, piperazine, thiophanate, chloralose, fenthion, pyridin-4-amine, strychnine, 1-hydroxy-1H-pyridine-2-thione, 4-(quinoxalin-2-ylamino)benzenesulfonamide, 8-hydroxyquinoline sulfate, bronopol, copper hydroxide, cresol, dipyrithione, dodicin, fenaminosulf, formaldehyde, hydrargaphen, kasugamycin, kasugamycin hydrochloride hydrate, nickel bis(dimethyldithiocarbamate), nitrapyrin, octhilinone, oxolinic acid, oxytetracycline, potassium hydroxyquinoline sulfate, probenazole, streptomycin, streptomycin sesquisulfate, tecloftalam, thiomersal, Adoxophyes orana GV,
Agrobacterium radiobacter, Amblyseius spp., Anagrapha falcifera NPV, Anagrus atomus, Aphelinus a bdominalis, Aphidius colemani, Aphidoletes aphidimyza, Autographa californica NPV,
Bacillus sphaericus Neide, Beauveria brongniartii, Chrysoperla carnea, Cryptolaemus montrouzieri, Cydia pomonella GV, Dacnusa sibirica, Diglyphus isaea, Encarsia formos a, Eretmocerus eremicus, Heterorhabditis bacteriophora and H. megidis, Hippodamia convergens, Leptomastix dactylopii, Macrolophus caliginosus, Mamestra brassicae NPV, Metaphycus helvolus, Metarhizium anisopliae var. acridum, Metarhizium anisopliae var. anisopliae, Ne odiprion sertifer NPV and N. lecontei NPV, Orius spp., Paecilomyces fumosoroseus, Phytoseiulus persimilis, Steinernema bibionis, Steinernema carpocapsae, Steinernema feltiae, Steiner nema glaseri, Steinernema riobrave, Steinernema riobravis, Steinernema scapterisci, Steinernema sp p., Trichogramma spp., Typhlodromus occidentalis, Verticillium lecanii, apholate, bisazir, busulfan, dimatif, hemel, hempa, metepa, methiotepa, methyl apholate, morzid, penfluron, tepa, thiohempa, thiotepa, tretamine, uredepa, (E)-dec-5-en-1-yl acetate with (E)-dec-5-en-1-ol, (E)-tridec-4-en-1-yl acetate, (E)-6-methylhept-2-en-4-ol, (E,Z)-tetradeca-4,10-dien-1-yl acetate, (Z)-dodec-7-en-1-yl acetate, (Z)-hexadec-11-enal, (Z)-hexadec-11-en-1-yl acetate, (Z)-hexadec-13-en-11-yn-1-yl acetate, (Z)-icos-13-en-10-one, (Z)-tetradec-7-en-1-al, (Z)-tetradec-9-en-1-ol, (Z)-tetradec-9-en-1-yl acetate, (7E,9Z)-dodeca-7,9-dien-1-yl acetate, (9Z,11E)-tetradeca-9,11-dien-1-yl acetate, (9Z,12E)-tetradeca-9,12-dien-1-yl acetate, 14-methyloctadec-1-ene, 4-methylnonan-5-ol with 4-methylnonan-5-one, alpha-multistriatin, brevicomin, codlelure, codlemone, cuelure, disparlure, dodec-8-en-1-yl acetate, dodec-9-en-1-yl acetate, dodeca-8, 10-dien-1-yl acetate, dominicalure, ethyl 4-methyloctanoate,
eugenol, frontalin, grandlure, grandlure I, grandlure II, grandlure III, grandlure IV, hexalure, ipsdienol, i psenol, japonilure, lineatin, litlure, looplure, medlure, megatomoic acid, methyl eugenol, muscalure, octadeca-2,13-dien-1-yl acetate, octadeca-3,13-dien-1-yl acetate, orfralure, oryctalure, ostramone, siglure, sordidin, sulcatol, tetradec-11-en-1-yl acetate, trimedlure, trimedlure A, trimedlure B₁, trimedlure B₂, trimedlure C, trunc-call, 2-(octylthio)-ethanol, butopyronoxyl, butoxy(polypropylene glycol), dibutyl adipate, dibutyl phthalate, dibutyl succinate, diethyltoluamide, dimethyl carbate, dimethyl phthalate, ethyl hexanediol, hexamide, methoquin-butyl, methylneodecanamide, oxamate, picaridin, 1-dichloro-1-nitroethane, 1,1-dichloro-2,2-bis(4-ethylphenyl)ethane, 1,2-dichloropropane with 1,3-dichloropropene, 1-bromo-2-chloroethane, 2,2,2-trichloro-1-(3,4-dichlorophenyl)ethyl acetate, 2,2-dichlorovinyl 2-ethylsulfinylethyl methyl phosphate, 2-(1,3-dithiolan-2-yl)phenyl dimethylcarbamate, 2-(2-butoxyethoxy)ethyl thiocyanate, 2-(4,5-dimethyl-1 ,3-dioxolan-2-yl)phenyl methylcarbamate, 2-(4-chloro-3,5-xylyloxy)ethanol, 2-chlorovinyl diethyl phosphate, 2-imidazolidone, 2-isovalerylindan-1,3-dione, 2-methyl(prop-2-ynyl)aminophenyl methylcarbamate, 2-thiocyanatoethyl laurate, 3-bromo-1-chloroprop-1-ene, 3-methyl-1-phenylpyrazol-5-yl dimethylcarbamate, 4-methyl(prop-2-ynyl)amino-3,5-xylyl methylcarbamate, 5,5-dimethyl-3-oxocyclohex-1-enyl dimethylcarbamate, acethion, acrylonitrile, aldrin, allosamidin, allyxycarb, alpha-ecdysone, aluminium phosphide, aminocarb, anabasine, athidathion, azamethiphos, Bacillus thuringiensis delta endotoxins, barium hexafluorosilicate, barium polysulfide, barthrin, Bayer 22/190, Bayer 22408, beta-cyfluthrin, beta-cypermethrin, bioethanomethrin, biopermethrin, bis(2-chloroethyl) ether, borax, bromfenvinfos, bromo-DDT, bufencarb, butacarb, butathiofos, butonate, calcium arsenate, calcium cyanide, carbon disulfide, carbon tetrachloride, cartap hydrochloride, cevadine, chlorbicyclen, chlordane, chlordecone, chloroform, chloropicrin, chlorphoxim, chlorprazophos, cis-resmethrin, cismethrin, clocythrin, copper acetoarsenite, copper arsenate, copper oleate, coumithoate, cryolite, CS 708, cyanofenphos, cyanophos, cyclethrin, cythioate, d-tetramethrin, DAEP, dazomet, decarbofuran, diamidafos, dicapthon, dichlofenthion, dicresyl, dicyclanil, dieldrin, diethyl 5-methylpyrazol-3-yl
phosphate, dilor, dimefluthrin, dimetan, dimethrin, dimethylvinphos, dimetilan, dinoprop, dinosam, dino seb, diofenolan, dioxabenzofos, dithicrofos, DSP, ecdysterone, EI 1642, EMPC, EPBP, etaphos, ethiofencarb, ethyl formate, ethylene dibromide, ethylene dichloride, ethylene oxide, EXD, fenchlorphos, fenethacarb, fenitrothion, fenoxacrim, fenpirithrin, fensulfothion, fenthion-ethyl, flucofuron, fosmethilan, fospirate, fosthietan, furathiocarb, furethrin, guazatine, guazatine acetates, sodium tetrathiocarbonate, halfenprox, HCH, HEOD, heptachlor, heterophos, HHDN, hydrogen cyanide, hyquincarb, IPSP, isazofos, isobenzan, isodrin, isofenphos, isolane, isoprothiolane, isoxathion, juvenile hormone I, juvenile hormone II, juvenile hormone III, kelevan, kinoprene, lead arsenate, leptophos, lirimfos, lythidathion, m-cumenyl methylcarbamate, magnesium phosphide, mazidox, mecarphon, menazon, mercurous chloride, mesulfenfos, metam, metam-potassium, metam-sodium, methanesulfonyl fluoride, methocrotophos, methoprene, methothrin, methoxychlor, methyl isothiocyanate, methylchloroform, methylene chloride, metoxadiazone, mirex, naftalofos, naphthalene, NC-170, nicotine, nicotine sulfate, nithiazine, nornicotine, O-5-dichloro-4-iodophenyl O-ethyl ethylphosphonothioate, O,O-diethyl O-4-methyl-2-oxo-2H-chromen-7-yl phosphorothioate, O,O-diethyl O-6-methyl-2-propylpyrimidin-4-yl phosphorothioate, O,O,O',O'-tetrapropyl dithiopyrophosphate, oleic acid, para-dichlorobenzene, parathion-methyl, pentachlorophenol, pentachlorophenyl laurate, PH 60-38, phenkapton, phosnichlor, phosphine, phoxim-methyl, pirimetaphos, polychlorodicyclopentadiene isomers, potassium arsenite, potassium thiocyanate, precocene I, precocene II, precocene III, primidophos, profluthrin, promecarb, prothiofos, pyrazophos, pyresmethrin, quassia, quinalphos-methyl, quinothion, rafoxanide, resmethrin, rotenone, kadethrin, ryania, ryanodine, sabadilla), schradan, sebufos, SI-0009, thiapronil, sodium arsenite, sodium cyanide, sodium fluoride, sodium hexafluorosilicate, sodium pentachlorophenoxide, sodium selenate, sodium thiocyanate, sulcofuron, sulcofuron-sodium, sulfuryl fluoride, sulprofos, tar oils, tazimcarb, TDE, tebupirimfos, temephos, terallethrin, tetrachloroethane, thicrofos, thiocyclam, thiocyclam hydrogen oxalate, thionazin, thiosultap, thiosultap-sodium, tralomethrin, transpermethrin, triazamate, trichlormetaphos-3, trichloronat, trimethacarb, tolprocarb, triclopyricarb, triprene, veratridine, veratrine, XMC, zetamethrin, zinc phosphide, zolaprofos, and meperfluthrin, tetramethylfluthrin, bis(tributyltin) oxide, bromoacetamide, ferric phosphate, niclosamide-olamine, tributyltin oxide, pyrimorph, trifenmorph, 1,2-dibromo-3-chloropropane, 1,3-dichloropropene, 3,4-dichlorotetrahydrothiophene 1,1-dioxide, 3-(4-chlorophenyl)-5-methylrhodanine, 5-methyl-6-thioxo-1,3,5-thiadiazinan-3-ylacetic acid, 6-isopentenylaminopurine, 2-fluoro-N-(3-methoxyphenyl)-9H-purin-6-amine, benclothiaz, cytokinins, DCIP, furfural, isamidofos, kinetin, Myrothecium verrucaria composition, tetrachlorothiophene, xylenols, zeatin, potassium ethylxanthate, acibenzolar, acibenzolar-S-methyl, Reynoutria sachalinensis extract, alpha-chlorohydrin, antu, barium carbonate, bisthiosemi, brodifacoum, bromadiolone, bromethalin, chlorophacinone, cholecalciferol, coumachlor, coumafuryl, coumatetralyl, crimidine, difenacoum, difethialone, diphacinone, ergocalciferol, flocoumafen, fluoroacetamide, flupropadine, flupropadine hydrochloride, norbormide, phosacetim, phosphorus, pindone, pyrinuron, scilliroside, sodium fluoroacetate, thallium sulfate, warfarin, 2-(2-butoxyethoxy)-ethyl piperonylate, 5-(1,3-benzodioxol-5-yl)-3-hexylcyclohex-2-enone, farnesol with nerolidol, verbutin, MGK 264, piperonyl butoxide, piprotal, propyl isomer, S421, sesamex, sesasmolin, sulfoxide, anthraquinone, copper naphthenate, copper oxychloride, dicyclopentadiene, thiram, zinc naphthenate, ziram, imanin, ribavirin, mercuric oxide, thiophanate-methyl, azaconazole, bitertanol, bromuconazole, cyproconazole, difenoconazole, diniconazole, epoxiconazole, fenbuconazole, fluquinconazole, flusilazole, flutriafol, furametpyr, hexaconazole, imazalil, imibenconazole, ipconazole, metconazole, myclobutanil, paclobutrazole, pefurazoate, penconazole, prothioconazole, pyrifenox, prochloraz, propiconazole, pyrisoxazole, simeconazole, tebuconazole, tetraconazole, triadimefon, triadimenol, triflumizole, triticonazole, ancymidol, fenarimol, nuarimol, bupirimate, dimethirimol, ethirimol, dodemorph, fenpropidin, fenpropimorph, spiroxamine, tridemorph, cyprodinil, mepanipyrim, pyrimethanil, fenpiclonil, fludioxonil, benalaxyl, furalaxyl, metalaxyl, R-metalaxyl, ofurace, oxadixyl, carbendazim, debacarb, fuberidazole, thiabendazole, chlozolinate, dichlozoline, myclozoline, procymi-done, vinclozoline, boscalid, carboxin, fenfuram, flutolanil, mepronil, oxycarboxin, penthiopyrad, thifluzamide, dodine, iminoctadine, azoxystrobin, dimoxystrobin, enestroburin, fenaminstrobin, flufenoxystrobin, fluoxastrobin, kresoxim-methyl, metominostrobin, trifloxystrobin, orysastrobin, picoxystrobin, pyraclostrobin, pyrametostrobin, pyraoxystrobin, ferbam, mancozeb, maneb, metiram, propineb, zineb, captafol, captan, fluoroimide, folpet, tolylfluanid, bordeaux mixture, copper oxide, mancopper, oxine-copper, nitrothal-isopropyl, edifenphos, iprobenphos, phosdiphen, tolclofos-methyl, anilazine, benthiavalicarb, blasticidin-S, chloroneb, chlorothalonil, cyflufenamid, cymoxanil, cyclobutrifluram, diclocymet, diclomezine, dicloran, diethofencarb, dimethomorph, flumorph, dithianon, ethaboxam, etridiazole, famoxadone, fenamidone, fenoxanil, ferimzone, fluazinam, fluopicolide, flusulfamide, fluxapyroxad, fenhexamid, fosetyl-aluminium, hymexazol, iprovalicarb, cyazofamid, methasulfocarb, metrafenone, pencycuron, phthalide, polyoxins, propamocarb, pyribencarb, proquinazid, pyroquilon, pyriofenone, quinoxyfen, quintozene, tiadinil, triazoxide, tricyclazole, triforine, validamycin, valifenalate, zoxamide, mandipropamid, flubeneteram, isopyrazam, sedaxane, benzovindiflupyr, pydiflumetofen, 3-difluoromethyl-1-methyl-1H-pyrazole-4-carboxylic acid (3',4',5'-trifluoro-biphenyl-2-yl)-amide, isoflucypram, isotianil, dipymetitrone, 6-ethyl-5,7-dioxo-pyrrolo[4,5][1,4]dithiino[1,2-c]isothiazole-3-carbonitrile, 2-(difluoromethyl)-N-[3-ethyl-1,1-dimethyl-indan-4-yl]pyridine-3-carboxamide, 4-(2,6-difluorophenyl)-6-methyl-5-phenyl-pyridazine-3-carbonitrile, (R)-3-(difluoromethyl)-1-methyl-N-[1,1,3-trimethylindan-4-yl]pyrazole-4-carboxamide, 4-(2-bromo-4-fluoro-phenyl)-N-(2-chloro-6-fluoro-phenyl)-2,5-dimethyl-pyrazol-3-amine, 4- (2- bromo- 4- fluorophenyl) - N- (2- chloro- 6- fluorophenyl) - 1, 3- dimethyl- 1H- pyrazol- 5- amine, fluindapyr, coumethoxystrobin (jiaxiangjunzhi), Ivbenmixianan, dichlobentiazox, mandestrobin, 3-(4,4-difluoro-3,4-dihydro-3,3-dimethylisoquinolin-1-yl)quinolone, 2-[2-fluoro-6-[(8-fluoro-2-methyl-3-quinolyl)oxy]phenyl]propan-2-ol, oxathiapiprolin, tert-butyl N-[6-[[[(1-methyltetrazol-5-yl)-phenylmethylene]amino]oxymethyl]-2-pyridyl]carbamate, pyraziflumid, inpyrfluxam, trolprocarb, mefentrifluconazole, ipfentrifluconazole, 2-(difluoromethyl)-N-[(3R)-3-ethyl-1,1-dimethyl-indan-4-yl]pyridine-3-carboxamide, N'-(2,5-dimethyl-4-phenoxy-phenyl)-N-ethyl-N-methyl-formamidine, N'-[4-(4,5-dichlorothiazol-2-yl)oxy-2,5-dimethyl-phenyl]-N-ethyl-N-methyl-formamidine, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chlorophenyl] methanesulfonate, but-3-ynyl N-[6-[[(Z)-[(1-methyltetrazol-5-yl)-phenylmethylene]amino]oxymethyl]-2-pyridyl]carbamate, methyl N-[[5-[4-(2,4-dimethylphenyl)triazol-2-yl]-2-methyl-phenyl]methyl]carbamate, 3-chloro-6-methyl-5-phenyl-4-(2,4,6-trifluorophenyl)pyridazine, pyridachlometyl, 3-(difluoromethyl)-1-methyl-N-[1,1,3-trimethylindan-4-yl]pyrazole-4-carboxamide, 1-[2-[[1-(4-chlorophenyl)pyrazol-3-yl]oxymethyl]-3-methyl-phenyl]-4-methyl-tetrazol-5-one, 1-methyl-4-[3-methyl-2-[[2-methyl-4-(3,4,5-trimethylpyrazol-1-yl)phenoxy]methyl]phenyl]tetrazol-5-one, aminopyrifen, ametoctradin, amisulbrom, penflufen, (Z,2E)-5-[1-(4-chlorophenyl)pyrazol-3-yl]oxy-2-methoxyimino-N,3-dimethyl-pent-3-enamide, florylpicoxamid, fenpicoxamid, tebufloquin, ipflufenoquin, quinofumelin, isofetamid, N-[2-[2,4-dichloro-phenoxy]phenyl]-3-(difluoromethyl)-1-methyl-pyrazole-4-carboxamide, N-[2-[2-chloro-4-(trifluoromethyl)phenoxy]phenyl]-3-(difluoromethyl)-1-methyl-pyrazole-4-carboxamide, benzothiostrobin, phenamacril, 5-amino-1,3,4-thiadiazole-2-thiol zinc salt (2:1), fluopyram, flutianil, fluopimomide, pyrapropoyne, picarbutrazox, 2-(difluoromethyl)-N-(3-ethyl-1,1-dimethyl-indan-4-yl)pyridine-3-carboxamide, 2- (difluoromethyl) - N- ((3R) - 1, 1, 3- trimethylindan- 4- yl) pyridine- 3- carboxamide, 4-[[6-[2-(2,4-difluorophenyl)-1,1-difluoro-2-hydroxy-3-(1,2,4-triazol-1-yl)propyl]-3-pyridyl]oxy]benzonitrile, metyltetraprole, 2- (difluoromethyl) - N- ((3R) - 1, 1, 3- trimethylindan- 4- yl) pyridine- 3- carboxamide, α- (1, 1- dimethylethyl) - α- [4'- (trifluoromethoxy) [1, 1'- biphenyl] - 4- yl] -5- pyrimidinemethanol, fluoxapiprolin, enoxastrobin, 4-[[6-[2-(2,4-difluorophenyl)-1,1-difluoro-2-hydroxy-3-(1,2,4-triazol-1-yl)propyl]-3-pyridyl]oxy] benzonitrile, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile, trinexapac, coumoxystrobin, zhongshengmycin, thiodiazole copper, zinc thiazole, amectotractin, iprodione, N-octyl-N'-[2-(octylamino)ethyl]ethane-1,2-diamine, N'-[5-bromo-2-methyl-6-[(1S)-1-methyl-2-propoxy-ethoxy]-3-pyridyl]-N-ethyl-N-methyl-formamidine, N'-[5-bromo-2-methyl-6-[(1R)-1-methyl-2-propoxy-ethoxy]-3-pyridyl]-N-ethyl-N-methyl-formamidine, N'-[5-bromo-2-methyl-6-(1-methyl-2-propoxy-ethoxy)-3-pyridyl]-N-ethyl-N-methyl-formamidine, N'-[5-chloro-2-methyl-6-(1-methyl-2-propoxy-ethoxy)-3-pyridyl]-N-ethyl-N-methyl-formamidine, N'-[5-bromo-2-methyl-6-(1-methyl-2-propoxy-ethoxy)-3-pyridyl]-N-isopropyl-N-methyl-formamidine (these compounds may be prepared from the methods described in WO2015/155075); N'-[5-bromo-2-methyl-6-(2-propoxypropoxy)-3-pyridyl]-N-ethyl-N-methyl-formamidine (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine, N'-[4-(1-cyclopropyl-2,2,2-trifluoro-1-hydroxyethyl)-5-methoxy-2-methyl-phenyl]-N-isopropyl-N-methyl-formamidine (these compounds may be prepared from the methods described in WO2018/228896); N-ethyl-N'-[5-methoxy-2-methyl-4-[2-trifluoromethyl)oxetan-2-yl]phenyl]-N-methyl-formamidine, N-ethyl-N'-[5-methoxy-2-methyl-4-[2-trifuoromethyl)tetrahydrofuran-2-yl]phenyl]-N-methyl-formamidine (these compounds may be prepared from the methods described in WO2019/110427); N-[(1R)-1-benzyl-3-chloro-1-methyl-but-3-enyl]-8-fluoro-quinoline-3-carboxamide, N-[(1S)-1-benzyl-3-chloro-1-methyl-but-3-enyl]-8-fluoro-quinoline-3-carboxamide, N-[(1R)-1-benzyl-3,3,3-trifluoro-1-methyl-propyl]-8-fluoro-quinoline-3-carboxamide, N-[(1S)-1-benzyl-3,3,3-trifluoro-1-methyl-propyl]-8-fluoro-quinoline-3-carboxamide, N-[(1R)-1-benzyl-1 ,3-dimethyl-butyl]-7,8-difluoro-quinoline-3-carboxamide,
N-[(1S)-1-benzyl-1,3-dimethyl-butyl]-7,8-difluoro-quinoline-3-carboxamide, 8-fluoro-N-[(1R)-1-[(3-fluorophenyl)methyl]-1 ,3-dimethyl-butyl]quinoline-3-carboxamide, 8-fluoro-N-[(1S)-1-[(3-fluorophenyl)methyl]-1,3-dimethyl-butyl]quinoline-3-carboxamide, N-[(1R)-1-benzyl-1,3-dimethyl-butyl]-8-fluoro-quinoline-3-carboxamide, N-[(1S)-1-benzyl-1,3-dimethyl-butyl]-8-fluoro-quinoline-3-carboxamide,
N-((1R)-1-benzyl-3-chloro-1-methyl-but-3-enyl)-8-fluoro-quinoline-3-carboxamide, N-((1S)-1-benzyl-3-chloro-1-methyl-but-3-enyl)-8-fluoro-quinoline-3-carboxamide (these compounds may be prepared from the methods described in WO2017/153380); 1-(6,7-dimethylpyrazolo[1,5-a]pyridin-3-yl)-4,4,5-trifluoro-3,3-dimethyl-isoquinoline, 1-(6,7-dimethylpyrazolo[1,5-a]pyridin-3-yl)-4,4,6-trifluoro-3,3-dimethyl-isoquinoline, 4,4-difluoro-3,3-dimethyl-1-(6-methylpyrazolo[1,5-a]pyridin-3-yl)isoquinoline, 4,4-difluoro-3,3-dimethyl-1-(7-methylpyrazolo[1 ,5-a]pyridin-3-yl)isoquinoline, 1-(6-chloro-7-methyl-pyrazolo[1,5-a]pyridin-3-yl)-4,4-difluoro-3,3-dimethyl-isoquinoline (these compounds may be prepared from the methods described in WO2017/025510); 1-(4,5-dimethylbenzimidazol-1-yl)-4,4,5-trifluoro-3,3-dimethyl-isoquinoline, 1-(4,5-dimethylbenzimidazol-1-yl)-4,4-difluoro-3,3-dimethyl-isoquinoline, 6-chloro-4,4-difluoro-3,3-dimethyl-1-(4-methylbenzimidazol-1-yl)isoquinoline, 4,4-difluoro-1-(5-fluoro-4-methyl-benzimidazol-1-yl)-3,3-dimethyl-isoquinoline, 3-(4,4-difluoro-3,3-dimethyl-1-isoquinolyl)-7,8-dihydro-6H-cyclopenta[e]benzimidazole (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide, N,2-dimethoxy-N-[[4-[5-(trifluoromethyl)-1,2,4-oxadiazol-3-yl]phenyl]methyl]propanamide, N-ethyl-2-methyl-N-[[4-[5-(trifluoromethyl)-1,2,4-oxadiazol-3-yl]phenyl]methyl]propanamide, 1-methoxy-3-methyl-1-[[4-[5-(trifluoromethyl)-1,2,4-oxadiazol-3-yl]phenyl]methyl]urea, 1,3-dimethoxy-1-[[4-[5-(trifluoromethyl)-1,2,4-oxadiazol-3-yl]phenyl]methyl]urea, 3-ethyl-1-methoxy-1-[[4-[5-(trifluoromethyl)-1,2,4-oxadiazol-3-yl]phenyl]methyl]urea, N-[[4-[5-(trifluoromethyl)-1,2,4-oxadiazol-3-yl]phenyl]methyl]propanamide, 4,4-dimethyl-2-[[4-[5-(trifluoromethyl)-1,2,4-oxadiazol-3-yl]phenyl]methyl]isoxazolidin-3-one, 5,5-dimethyl-2-[[4-[5-(trifluoromethyl)-1,2,4-oxadiazol-3-yl]phenyl]methyl]isoxazolidin-3-one, ethyl 1-[[4-[5-(trifluoromethyl)-1,2,4-oxadiazol-3-yl]phenyl]methyl]pyrazole-4-carboxylate, N,N-dimethyl-1-[[4-[5-(trifluoromethyl)-1,2,4-oxadiazol-3-yl]phenyl]methyl]-1,2,4-triazol-3-amine (these compounds may be prepared from the methods described in WO 2017/055473, WO 2017/055469, WO 2017/093348 and WO 2017/118689); 2-[6-(4-chlorophenoxy)-2-(trifluoromethyl)-3-pyridyl]-1-(1,2,4-triazol-1-yl)propan-2-ol
(this compound may be prepared from the methods described in WO 2017/029179); 2-[6-(4-bromophenoxy)-2-(trifluoromethyl)-3-pyridyl]-1-(1,2,4-triazol-1-yl)propan-2-ol (this compound may be prepared from the methods described in WO 2017/029179); 3-[2-(1-chlorocyclopropyl)-3-(2-fluorophenyl)-2-hydroxy-propyl]imidazole-4-carbonitrile (this compound may be prepared from the methods described in WO 2016/156290); 3-[2-(1-chlorocyclopropyl)-3-(3-chloro-2-fluoro-phenyl)-2-hydroxy-propyl]imidazole-4-carbonitrile (this compound may be prepared from the methods described in WO 2016/156290); (4-phenoxyphenyl)methyl 2-amino-6-methyl-pyridine-3-carboxylate (this compound may be prepared from the methods described in WO 2014/006945); 2,6-Dimethyl-1H,5H-[1,4]dithiino[2,3-c:5,6-c']dipyrrole-1,3,5,7(2H,6H)-tetrone (this compound may be prepared from the methods described in WO 2011/138281) N-methyl-4-[5-(trifluoromethyl)-1,2,4-oxadiazol-3-yl]benzenecarbothioamide; N-methyl-4-[5-(trifluoromethyl)-1,2,4-oxadiazol-3-yl]benzamide; (Z,2E)-5-[1-(2,4-dichlorophenyl)pyrazol-3-yl]oxy-2-methoxyimino-N,3-dimethyl-pent-3-enamide (this compound may be prepared from the methods described in WO 2018/153707); N'-(2-chloro-5-methyl-4-phenoxy-phenyl)-N-ethyl-N-methyl-formamidine; N'-[2-chloro-4-(2-fluorophenoxy)-5-methyl-phenyl]-N-ethyl-N-methyl-formamidine (this compound may be prepared from the methods described in WO 2016/202742); 2-(difluoromethyl)-N-[(3S)-3-ethyl-1,1-dimethyl-indan-4-yl]pyridine-3-carboxamide (this compound may be prepared from the methods described in WO 2014/095675); (5-methyl-2-pyridyl)-[4-[5-(trifluoromethyl)-1,2,4-oxadiazol-3-yl]phenyl]methanone, (3-methylisoxazol-5-yl)-[4-[5-(trifluoromethyl)-1,2,4-oxadiazol-3-yl]phenyl]methanone (these compounds may be prepared from the methods described in WO 2017/220485); 2-oxo-N-propyl-2-[4-[5-(trifluoromethyl)-1,2,4-oxadiazol-3-yl]phenyl]acetamide (this compound may be prepared from the methods described in WO 2018/065414); ethyl 1-[[5-[5-(trifluoromethyl)-1,2,4-oxadiazol-3-yl]-2-thienyl]methyl]pyrazole-4-carboxylate (this compound may be prepared from the methods described in WO 2018/158365); 2,2-difluoro-N-methyl-2-[4-[5-(trifluoromethyl)-1,2,4-oxadiazol-3-yl]phenyl]acetamide, N-[(E)-methoxyiminomethyl]-4-[5-(trifluoromethyl)-1,2,4-oxadiazol-3-yl]benzamide, N-[(Z)-methoxyiminomethyl]-4-[5-(trifluoromethyl)-1,2,4-oxadiazol-3-yl]benzamide, N-[N-methoxy-C-methyl-carbonimidoyl]-4-[5-(trifluoromethyl)-1,2,4-oxadiazol-3-yl]benzamide (these compounds may be prepared from the methods described in WO 2018/202428), seboctylamine, chloroinconazide, flumetylsulforim, fluoxytioconazole, flufenoxadiazam, metarylpicoxamid.

The compounds of the invention may also be used in combination with anthelmintic agents. Such anthelmintic agents include, compounds selected from the macrocyclic lactone class of compounds such as ivermectin, avermectin, abamectin, emamectin, eprinomectin, doramectin, selamectin, moxidectin, nemadectin and milbemycin derivatives as described in EP- 357460, EP-444964 and EP-594291. Additional anthelmintic agents include semisynthetic and biosynthetic avermectin/milbemycin derivatives such as those described in US-5015630, WO-9415944 and WO-9522552. Additional anthelmintic agents include the benzimidazoles such as albendazole, cambendazole, fenbendazole, flubendazole, mebendazole, oxfendazole, oxibendazole, parbendazole, and other members of the class. Additional anthelmintic agents include imidazothiazoles and tetrahydropyrimidines such as tetramisole, levamisole, pyrantel pamoate, oxantel or morantel. Additional anthelmintic agents include flukicides, such as triclabendazole and clorsulon and the cestocides, such as praziquantel and epsiprantel.

The compounds of the invention may be used in combination with derivatives and analogues of the paraherquamide/marcfortine class of anthelmintic agents, as well as the antiparasitic oxazolines such as those disclosed in US-5478855, US- 4639771 and DE-19520936.

The compounds of the invention may be used in combination with derivatives and analogues of the general class of dioxomorpholine antiparasitic agents as described in WO 96/15121 and also with anthelmintic active cyclic depsipeptides such as those described in WO 96/11945, WO 93/19053, WO 93/25543, EP 0 626 375, EP 0 382 173, WO 94/19334, EP 0 382 173, and EP 0 503 538.

The compounds of the invention may be used in combination with other ectoparasiticides; for example, fipronil; pyrethroids; organophosphates; insect growth regulators such as lufenuron; ecdysone agonists such as tebufenozide and the like; neonicotinoids such as imidacloprid and the like.

The compounds of the invention may be used in combination with terpene alkaloids, for example those described in International Patent Application Publication Numbers WO 95/19363 or WO 04/72086, particularly the compounds disclosed therein.

Other examples of such biologically active compounds that the compounds of the invention may be used in combination with include but are not restricted to the following:
Organophosphates: acephate, azamethiphos, azinphos-ethyl, azinphos- methyl, bromophos, bromophos-ethyl, cadusafos, chlorethoxyphos, chlorpyrifos, chlorfenvinphos, chlormephos, demeton, demeton-S-methyl, demeton-S-methyl sulphone, dialifos, diazinon, dichlorvos, dicrotophos, dimethoate, disulfoton, ethion, ethoprophos, etrimfos, famphur, fenamiphos, fenitrothion, fensulfothion, fenthion, flupyrazofos, fonofos, formothion, fosthiazate, heptenophos, isazophos, isothioate, isoxathion, malathion, methacriphos, methamidophos, methidathion, methyl-parathion, mevinphos, monocrotophos, naled, omethoate, oxydemeton-methyl, paraoxon, parathion, parathion-methyl, phenthoate, phosalone, phosfolan, phosphocarb, phosmet, phosphamidon, phorate, phoxim, pirimiphos, pirimiphos-methyl, profenofos, propaphos, proetamphos, prothiofos, pyraclofos, pyridapenthion, quinalphos, sulprophos, temephos, terbufos, tebupirimfos, tetrachlorvinphos, thimeton, triazophos, trichlorfon, vamidothion.

Carbamates: alanycarb, aldicarb, 2-sec-butylphenyl methylcarbamate, benfuracarb, carbaryl, carbofuran, carbosulfan, cloethocarb, ethiofencarb, fenoxycarb, fenthiocarb, furathiocarb, HCN-801, isoprocarb, indoxacarb, methiocarb, methomyl, 5-methyl-m-cumenylbutyryl(methyl)carbamate, oxamyl, pirimicarb, propoxur, thiodicarb, thiofanox, triazamate, UC-51717.

Pyrethroids: acrinathin, allethrin, alphametrin, 5-benzyl-3-furylmethyl (E)-(1 R)-cis-2,2-dimethyl-3-(2-oxothiolan-3-ylidenemethyl)cyclopropanecarboxylate, bifenthrin, beta-cyfluthrin, cyfluthrin, a-cypermethrin, beta-cypermethrin, bioallethrin, bioallethrin((S)-cyclopentylisomer), bioresmethrin, bifenthrin, NCI-85193, cycloprothrin, cyhalothrin, cythithrin, cyphenothrin, deltamethrin, empenthrin, esfenvalerate, ethofenprox, fenfluthrin, fenpropathrin, fenvalerate, flucythrinate, flumethrin, fluvalinate (D isomer), imiprothrin, cyhalothrin, lambda-cyhalothrin, permethrin, phenothrin, prallethrin, pyrethrins (natural products), resmethrin, tetramethrin, transfluthrin, theta-cypermethrin, silafluofen, t-fluvalinate, tefluthrin, tralomethrin, Zeta-cypermethrin.

Arthropod growth regulators: a) chitin synthesis inhibitors: benzoylureas: chlorfluazuron, diflubenzuron, fluazuron, flucycloxuron, flufenoxuron, hexaflumuron, lufenuron, novaluron, teflubenzuron, triflumuron, buprofezin, diofenolan, hexythiazox, etoxazole, chlorfentazine; b) ecdysone antagonists: halofenozide, methoxyfenozide, tebufenozide; c) juvenoids: pyriproxyfen, methoprene (including S-methoprene), fenoxycarb; d) lipid biosynthesis inhibitors: spirodiclofen.

Other antiparasitics: acequinocyl, amitraz, AKD-1022, ANS-118, azadirachtin, Bacillus thuringiensis, bensultap, bifenazate, binapacryl, bromopropylate, BTG-504, BTG-505, camphechlor, cartap, chlorobenzilate, chlordimeform, chlorfenapyr, chromafenozide, clothianidine, cyromazine, diacloden, diafenthiuron, DBI-3204, dinactin, dihydroxymethyldihydroxypyrrolidine, dinobuton, dinocap, endosulfan, ethiprole, ethofenprox, fenazaquin, flumite, MTI- 800, fenpyroximate, fluacrypyrim, flubenzimine, flubrocythrinate, flufenzine, flufenprox, fluproxyfen, halofenprox, hydramethylnon, IKI-220, kanemite, NC-196, neem guard, nidinorterfuran, nitenpyram, SD-35651, WL-108477, pirydaryl, propargite, protrifenbute, pymethrozine, pyridaben, pyrimidifen, NC-1111, R-195,RH-0345, RH-2485, RYI-210, S-1283, S-1833, SI-8601, silafluofen, silomadine, spinosad, tebufenpyrad, tetradifon, tetranactin, thiacloprid, thiocyclam, thiamethoxam, tolfenpyrad, triazamate, triethoxyspinosyn, trinactin, verbutin, vertalec, YI-5301.

Biological agents: Bacillus thuringiensis ssp aizawai, kurstaki, Bacillus thuringiensis delta endotoxin, baculovirus, entomopathogenic bacteria, virus and fungi.

Bactericides: chlortetracycline, oxytetracycline, streptomycin.

Other biological agents: enrofloxacin, febantel, penethamate, moloxicam, cefalexin, kanamycin, pimobendan, clenbuterol, omeprazole, tiamulin, benazepril, pyriprole, cefquinome, florfenicol, buserelin, cefovecin, tulathromycin, ceftiour, carprofen, metaflumizone, praziquarantel, triclabendazole.

The following mixtures of the compounds of Formula (I) with active ingredients are preferred. The abbreviation "TX" means one compound selected from the group consisting of the compounds as represented in Tables A1 to A23, B1 to B23, C1 to C23 and D1 to D23 (above) or Table E (below):
a compound selected from the group of substances consisting of petroleum oils + TX, 1,1 -bis(4-chlorophenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxafos + TX, benzyl benzoate + TX, bixafen + TX, brofenvalerate + TX, bromocyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chino-methionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dinopenton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fenpyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methylpropyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX , Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8 + TX, 10-dien-1 -yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)ethane + TX, 1,2-dichloropropane with 1,3-dichloropropene + TX, 1-bromo-2-chloroethane + TX, 2,2,2-trichloro-1-(3,4-dichlorophenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethylcarbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, EI 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl O-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla) + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, and meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1 ,2-dibromo-3-chloropropane + TX, 1 ,3-dichloropropene + TX, 3,4-dichlorotetrahydrothiophene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, 2-fluoro-N-(3-methoxyphenyl)-9H-purin-6-amine + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX ,acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, sodium fluoroacetate + TX, thallium sulfate + TX, warfarin + TX, 2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, mercuric oxide + TX, thiophanate-methyl + TX, azaconazole + TX, bitertanol + TX, bromuconazole + TX, cyproconazole + TX, difenoconazole + TX, diniconazole + TX, epoxiconazole + TX, fenbuconazole + TX, fluquinconazole + TX, flusilazole + TX, flutriafol + TX, furametpyr + TX, hexaconazole + TX, imazalil + TX, imiben-conazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, simeconazole + TX, tebuconazole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidin + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, metalaxyl -+ TX, Rmetalaxyl + TX, ofurace + TX, oxadixyl + TX, carbendazim + TX, debacarb + TX, fuberidazole + TX, thiabendazole + TX, chlozolinate + TX, dichlozoline + TX, myclozoline + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoxim-methyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb + TX, chlorothalonil + TX, cyflufenamid + TX, cymoxanil + TX, cyclobutrifluram + TX, diclocymet + TX, diclomezine + TX, dicloran + TX, diethofencarb + TX, dimethomorph + TX, flumorph + TX, dithianon + TX, ethaboxam + TX, etridiazole + TX, famoxadone + TX, fenamidone + TX, fenoxanil + TX, ferimzone + TX, fluazinam + TX, fluopicolide + TX, flusulfamide + TX, fluxapyroxad + TX, fenhexamid + TX, fosetyl-aluminium + TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluoro-phenyl)-2,5-dimethyl-pyrazol-3-amine + TX, 4-(2- bromo- 4- fluorophenyl) - N- (2- chloro- 6- fluorophenyl) - 1, 3- dimethyl- 1H- pyrazol- 5- amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate + TX, but-3-ynyl N-[6-[[(Z)-[(1-methyltetrazol-5-yl)-phenyl-methylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, tebufloquin + TX, ipflufenoquin + TX, quinofumelin + TX, isofetamid + TX, N-[2-[2,4-dichloro-phenoxy]phenyl]-3-(difluoromethyl)-1-methyl-pyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) - N- ((3R) - 1, 1, 3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2-(difluoromethyl) - N- ((3R) - 1, 1, 3- trimethylindan- 4- yl) pyridine- 3- carboxamide + TX, α- (1, 1-dimethylethyl) - α- [4'- (trifluoromethoxy) [1, 1'- biphenyl] - 4- yl] -5- pyrimidinemethanol + TX, fluoxapiprolin + TX, enoxastrobin + TX, 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, N-octyl-N'-[2-(octylamino)ethyl]ethane-1,2-diamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxy-ethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[2-trifluoromethyl)oxetan-2-yl]phenyl]-N-methyl-formamidine + TX, N-ethyl-N'-[5-methoxy-2-methyl-4-[2-trifuoromethyl)tetrahydrofuran-2-yl]phenyl]-N-methyl-formamidine + TX (these compounds may be prepared from the methods described in WO2019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methylpropyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380);
1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX. The compounds in this paragraph may be prepared from the methods described in WO 2017/055473, WO 2017/055469, WO 2017/093348 and WO 2017/118689; 2-[6-(4-chlorophenoxy)-2-(trifluoromethyl)-3-pyridyl]-1-(1,2,4-triazol-1-yl)propan-2-ol + TX (this compound may be prepared from the methods described in WO 2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO 2017/029179); 3-[2-(1-chlorocyclopropyl)-3-(2-fluorophenyl)-2-hydroxy-propyl]imidazole-4-carbonitrile + TX (this compound may be prepared from the methods described in WO 2016/156290); 3-[2-(1-chlorocyclopropyl)-3-(3-chloro-2-fluoro-phenyl)-2-hydroxy-propyl]imidazole-4-carbonitrile + TX (this compound may be prepared from the methods described in WO 2016/156290); (4-phenoxyphenyl)methyl 2-amino-6-methyl-pyridine-3-carboxylate + TX (this compound may be prepared from the methods described in WO 2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO 2011/138281); N-methyl-4-[5-(trifluoromethyl)-1,2,4-oxadiazol-3-yl]benzenecarbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO 2018/153707); N'-(2-chloro-5-methyl-4-phenoxy-phenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO 2016/202742); 2-(difluoromethyl)-N-[(3S)-3-ethyl-1,1-dimethyl-indan-4-yl]pyridine-3-carboxamide + TX (this compound may be prepared from the methods described in WO 2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO 2017/220485); 2-oxo-N-propyl-2-[4-[5-(trifluoromethyl)-1,2,4-oxadiazol-3-yl]phenyl]acetamide + TX (this compound may be prepared from the methods described in WO 2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO 2018/158365) ; 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO 2018/202428), seboctylamine + TX, chloroinconazide + TX, flumetylsulforim + TX, fluoxytioconazole + TX, flufenoxadiazam + TX, metarylpicoxamid + TX.

The references in brackets behind the active ingredients, e.g. [3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright ^{©} 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula (I) selected from one compound as represented in Tables A1 to A23, B1 to B23, C1 to C23 and D1 to D23 (above) or Table E (below) is preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound as represented in Tables A1 to A23, B1 to B23, C1 to C23 and D1 to D23 (above) or Table E (below), and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying a compound as represented in Tables A1 to A23, B1 to B23, C1 to C23 and D1 to D23 (above) or Table E (below) and the active ingredient(s) as described above, is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds (I) for the preparation of these compositions are also a subject of the invention.

Another aspect of the invention is related to the use of a compound of Formula (I) or of a preferred individual compound as defined herein, of a composition comprising at least one compound of Formula (I) or at least one preferred individual compound as above-defined, or of a fungicidal or insecticidal mixture comprising at least one compound of Formula (I) or at least one preferred individual compound as above-defined, in admixture with other fungicides or insecticides as described above, for controlling or preventing infestation of plants, e.g. useful plants such as crop plants, propagation material thereof, e.g. seeds, harvested crops, e.g. harvested food crops, or non-living materials by insects or by phytopathogenic microorganisms, preferably fungal organisms..

A further aspect of invention is related to a method of controlling or preventing an infestation of plants, e.g. useful plants such as crop plants, propagation material thereof, e.g. seeds, harvested crops, e.g. harvested food crops, or of non-living materials by phytopathogenic or spoilage microorganisms or organisms potentially harmful to man, especially fungal organisms, which comprises the application of a compound of formula (I) or of a preferred individual compound as above-defined as active ingredient to the plants, to parts of the plants or to the locus thereof, to the propagation material thereof, or to any part of the non-living materials.

Controlling or preventing means reducing infestation by insects or by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, or insects which comprises the application of a compound of formula (I), or an agrochemical composition which contains at least one of said compounds, is foliar application. The frequency of application and the rate of application will depend on the risk of infestation by the corresponding pathogen or insect. However, the compounds of formula (I) can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula (I) may also be applied to seeds (coating) by impregnating the seeds or tubers either with a liquid formulation of the fungicide or coating them with a solid formulation.

A formulation, e.g. a composition containing the compound of formula (I), and, if desired, a solid or liquid adjuvant or monomers for encapsulating the compound of formula (I), may be prepared in a known manner, typically by intimately mixing and/or grinding the compound with extenders, for example solvents, solid carriers and, optionally, surface active compounds (surfactants).

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is preferably 1g to 2000 g of active ingredient per hectare, more preferably 10 to 1000 g/ha, most preferably 10 to 600 g/ha. When used as seed drenching agent, convenient dosages are from 10mg to 1g of active substance per kg of seeds.

When the combinations of the present invention are used for treating seed, rates of 0.001 to 50 g of a compound of formula (I) per kg of seed, preferably from 0.01 to 10g per kg of seed are generally sufficient.

Suitably, a composition comprising a compound of formula (I) according to the present invention is applied either preventative, meaning prior to disease development or curative, meaning after disease development.

The compositions of the invention may be employed in any conventional form, for example in the form of a twin pack, a powder for dry seed treatment (DS), an emulsion for seed treatment (ES), a flowable concentrate for seed treatment (FS), a solution for seed treatment (LS), a water dispersible powder for seed treatment (WS), a capsule suspension for seed treatment (CF), a gel for seed treatment (GF), an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP) or any technically feasible formulation in combination with agriculturally acceptable adjuvants.

Such compositions may be produced in conventional manner, e.g. by mixing the active ingredients with appropriate formulation inerts (diluents, solvents, fillers and optionally other formulating ingredients such as surfactants, biocides, anti-freeze, stickers, thickeners and compounds that provide adjuvancy effects). Also conventional slow release formulations may be employed where long lasting efficacy is intended. Particularly formulations to be applied in spraying forms, such as water dispersible concentrates (e.g. EC, SC, DC, OD, SE, EW, EO and the like), wettable powders and granules, may contain surfactants such as wetting and dispersing agents and other compounds that provide adjuvancy effects, e.g. the ondensation product of formaldehyde with naphthalene sulphonate, an alkylarylsulphonate, a lignin sulphonate, a fatty alkyl sulphate, and ethoxylated alkylphenol and an ethoxylated fatty alcohol.

A seed dressing formulation is applied in a manner known per se to the seeds employing the combination of the invention and a diluent in suitable seed dressing formulation form, e.g. as an aqueous suspension or in a dry powder form having good adherence to the seeds. Such seed dressing formulations are known in the art. Seed dressing formulations may contain the single active ingredients or the combination of active ingredients in encapsulated form, e.g. as slow release capsules or microcapsules.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula (I) together with component (B) and (C), and optionally other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

Whereas it is preferred to formulate commercial products as concentrates, the end user will normally use dilute formulations.

### EXAMPLES

The Examples which follow serve to illustrate the invention.

Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm, 0.8 ppm or 0.2 ppm.

Compounds of Formula (I) may possess any number of benefits including, *inter alia,* advantageous levels of biological activity for protecting plants against diseases that are caused by fungi or superior properties for use as agrochemical active ingredients (for example, greater biological activity, an advantageous spectrum of activity, an increased safety profile (including improved crop tolerance), improved physico-chemical properties, or increased biodegradability).

Throughout this description, temperatures are given in degrees Celsius and "m.p." means melting point. LC/MS means Liquid Chromatography Mass Spectroscopy and the description of the apparatus and the methods are described below.

### Formulation Examples

| Wettable powders | a) | b) | c) |
|---|---|---|---|
| active ingredient [compound of formula (I)] | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether | - | 2 % | - |
| (7-8 mol of ethylene oxide) | | | |
| highly dispersed silicic acid | 5 % | 10 % | 10 % |
| Kaolin | 62 % | 27 % | - |

The active ingredient is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredient [compound of formula (I)] | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 |

The active ingredient is thoroughly mixed with the adjuvants and the mixture is thoroughly ground in a suitable mill, affording powders that can be used directly for seed treatment.

### Emulsifiable concentrate

| | |
|---|---|
| active ingredient [compound of formula (I)] | 10 % |
| octylphenol polyethylene glycol ether | 3 % |
| (4-5 mol of ethylene oxide) | |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredient [compound of formula (I)] | 5 % | 6 % | 4 % |
| talcum | 95 % | - | - |
| Kaolin | - | 94 % | - |
| mineral filler | - | - | 96 % |

Ready-for-use dusts are obtained by mixing the active ingredient with the carrier and grinding the mixture in a suitable mill. Such powders can also be used for dry dressings for seed.

### Extruder granules

| | |
|---|---|
| Active ingredient [compound of formula (I)] | 15 % |
| sodium lignosulfonate | 2 % |
| carboxymethylcellulose | 1 % |
| Kaolin | 82 % |

The active ingredient is mixed and ground with the adjuvants, and the mixture is moistened with water. The mixture is extruded and then dried in a stream of air.

### Coated granules

| | |
|---|---|
| Active ingredient [compound of formula (I)] | 8 % |
| polyethylene glycol (mol. wt. 200) | 3 % |
| Kaolin | 89 % |

The finely ground active ingredient is uniformly applied, in a mixer, to the kaolin moistened with polyethylene glycol. Non-dusty coated granules are obtained in this manner.

### Suspension concentrate

| | |
|---|---|
| active ingredient [compound of formula (I)] | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredient [compound of formula (I)] | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of a combination of the compound of formula (I) are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed.

The obtained capsule suspension is stabilized by adding 0.25 parts of a thickener and 3 parts of a dispersing agent. The capsule suspension formulation contains 28% of the active ingredients. The medium capsule diameter is 8-15 microns.

The resulting formulation is applied to seeds as an aqueous suspension in an apparatus suitable for that purpose.

### Preparation examples

### List of Abbreviations:

- Aq: = aqueous
- Ar: = argon
- *br s*: *= broad singlet*
- °C: = degrees Celsius
- DCM: = dichloromethane
- *dd*: *= doublet of doublet*
- DMF: = dimethylformamide
- *d*: *= doublet*
- EtOAc: = ethyl acetate
- equ.: = equivalent
- h: = hour(s)
- *M*: *= molar*
- *m*: = *multiplet*
- *min*: = *minutes*
- *MHz*: = *megahertz*
- mp: = melting point
- *ppm*: = *parts per million*
- *RT*: = *room temperature*
- *Rₜ*: = *retention time*
- *s*: = *singlet*
- *t*: = *triplet*
- THF: = tetrahydrofuran
- LC/MS: = Liquid Chromatography Mass Spectrometry (description of the apparatus and the methods used for LC/MS analysis are given above)

### Preparation examples:

### Example A1: 4-[(4-fluorophenoxy)methyl]-2-(8-fluoro-3-quinolyl)-4,6,6-trimethyl-5H-1,3-oxazine (compound E.04)

### Step 1:

To a solution of 4-fluorophenol (3.0 g, 26.5 mmol) in acetone (50 mL) at RT was added potassium carbonate (4.39 g, 31.8 mmol, 1.2 equ.), potassium iodide (0.44 g, 2.65 mmol, 0.1 equ.) and chloroacetone (2.66 mL, 31.8 mmol, 1.2 equ.). The mixture was warmed to 50°C and was stirred at 50°C for 2 h. The reaction mixture was then cooled to RT and partitioned between EtOAc and water. The organic phase was washed with brine, dried over MgSO₄, filtered and concentrated. The residue was purified by flash chromatography (silica gel, cyclohexane: EtOAc) to give 1-(4-fluorophenoxy)propan-2-one as a colorless liquid.

LC-MS (Method G), Rt = 0.79 min.

¹H NMR (400 MHz, CDCl₃) δ ppm: 6.96-7.06 (m, 2H), 6.80-6.91 (m, 2H), 4.53 (s, 2H), 2.29 (s, 3H).

### Step 2:

To a solution of 1-(4-fluorophenoxy)propan-2-one (4.30 g, 25.6 mmol) in THF (40 mL) was added titanium ethoxide (8.20 mL, 33.2 mmol, 1.3 equ.) and 2-methylpropane-2-sulfinamide (3.83 g, 30.7 mmol, 1.2 equ.). The resulting solution was warmed to 60°C and stirred at 60°C for 2 h. The reaction mixture was cooled to RT and quenched by addition of NaHCO₃ solution. The resulting suspension was filtered over a pad of celite and the filter cake was washed with EtOAc. The filtrate was extracted with EtOAc, the organic phase was washed with brine, dried over MgSO₄, filtered and concentrated. The residue was purified by flash chromatography (silica gel, cyclohexane: EtOAc) to give N-[2-(4-fluorophenoxy)-1-methyl-ethylidene]-2-methyl-propane-2-sulfinamide as a colorless liquid.

LC-MS (Method G), Rt = 0.97 min, (M+H) = 272.

¹H NMR (400MHz, CDCl₃) δ ppm: 6.94-7.05 (m, 2H), 6.81-6.91 (m, 2H), 4.47-4.67 (m, 2H), 2.42 (s, 3H), 1.20-1.23 (s, 9H).

### Step 3:

To 2-methylallyl magnesium chloride (0.5 M in THF, 20 mL, 10.1mmol, 1.5 equ.), cooled to - 50°C under argon, was added dropwise a solution of N-[2-(4-fluorophenoxy)-1-methyl-ethylidene]-2-methyl-propane-2-sulfinamide (2.60 g, 6.71 mmol) in DCM (20 mL). The resulting solution was gradually warmed from -50°C to -10°C over 3 h. Additional 2-methylallyl magnesium chloride (0.5 M in THF, 20 mL, 10.1 mmol, 1.5 equ.) was added and the reaction was gradually warmed to RT over 2 h. The reaction mixture was then quenched by addition of NH₄Cl solution and was extracted with EtOAc. The organic phase was washed with brine, dried over MgSO₄, filtered and concentrated. The residue was purified by flash chromatography (silica gel, cyclohexane: EtOAc) to give N-[1-[(4-fluorophenoxy)methyl]-1,3-dimethyl-but-3-enyl]-2-methyl-propane-2-sulfinamide (mixture of diastereoisomers) as yellow liquid.

LC-MS (Method G), Rt = 1.14 min, (M+H) = 328.

### Step 4:

To a solution of N-[1-[(4-fluorophenoxy)methyl]-1,3-dimethyl-but-3-enyl]-2-methyl-propane-2-sulfinamide (1.08 g, 3.3 mmol) in methanol (5 mL), cooled to 5°C, was added HCl (4M in dioxane) (1.6 mL, 6.6mmol, 2 equ.) and the solution was stirred for 2 h at 0°C. The reaction mixture was then concentrated under reduce pressure to give a mixture of 1-(4-fluorophenoxy)-2,4-dimethyl-pent-4-en-2-amine hydrochloride and methyl 2-methylpropane-2-sulfinate was used without further purification for the next step.

LC-MS (Method G), Rt = 0.69 min, (M+H) = 224.

### Step 5:

To a solution of 8-fluoroquinoline-3-carboxylic acid (0.650 g, 3.4 mmol) and 1-(4-fluorophenoxy)-2,4-dimethyl-pent-4-en-2-amine hydrochloride (0.88 g, 3.4 mmol, 1 equ.) in acetonitrile (14 mL) at RT was added triethylamine (1.43 mL, 10.2 mmol, 3 equ.) and propanephosphonic acid anhydride (50 % in EtOAc, 3.24 mL, 5.44 mmol, 1.6 equ.). The resulting solution was stirred for 2 h at RT, diluted with EtOAc and quenched with water. The mixture was extracted with EtOAc, the organic phase was washed with brine, dried over MgSO₄, filtered and concentrated. The residue was purified by flash chromatography (silica gel: cyclohexane: EtOAc) to give 8-fluoro-N-[1-[(4-fluorophenoxy)methyl]-1,3-dimethyl-but-3-enyl]quinoline-3-carboxamide as yellow gum.

LC-MS (Method G), Rt = 1.15 min, (M+H) = 397.

¹H NMR (400 MHz, CDCl₃) δ ppm: 9.26 (d, 1H), 8.58 (s, 1H), 7.73 (d, 1H), 7.44-7.63 (m, 2H), 6.86-7.06 (m, 4H), 6.53 (s, 1H), 5.03-5.07 (m, 1H), 4.88-4.93 (m, 1H), 4.31 (d, 1H), 4.17 (d, 1H), 2.92 (d, 1H), 2.64 (d, 1H), 1.89 (s, 3H), 1.69 (s, 3H).

¹⁹F NMR (CDCl₃) δ ppm: -123.23 (s, 1F), -124.61 (s, 1F).

### Step 6:

To a solution of 8-fluoro-N-[1-[(4-fluorophenoxy)methyl]-1,3-dimethyl-but-3-enyl]quinoline-3-carboxamide (0.670 g, 1.69 mmol) in acetonitrile (8 mL) was added trifluoroacetic acid (1.30 mL, 16.9 mmol, 10 equ.) at RT, the resulting solution was warmed to 80°C and stirred at 80°C for 20 h. The reaction mixture was then cooled to RT, diluted with EtOAc and carefully quenched with aq. NaHCO₃ solution. The neutralized mixture (pH > 8) was extracted with EtOAc, the organic phase was washed with brine, dried over MgSO₄, filtered and concentrated. The residue was purified by flash chromatography (silica gel, cyclohexane: EtOAc) to give 4-[(4-fluorophenoxy)methyl]-2-(8-fluoro-3-quinolyl)-4,6,6-trimethyl-5H-1,3-oxazine as yellow gum.

LC-MS (Method G), Rt = 1.18 min, (M+H) = 397.

¹H NMR (400 MHz, CDCl₃) δ ppm: 9.51 (d, 1H), 8.68 (br s, 1H), 7.70 (d, 1H), 7.43-7.55 (m, 2H), 6.95-7.02 (m, 2H), 6.85-6.95 (m, 2H), 3.95 (d, 1H), 3.82 (d, 1H), 2.23 (d, 1H), 1.97 (d, 1H), 1.53-1.57 (d, 6H), 1.51-1.53 (s, 3H).

¹⁹F NMR (CDCl₃) δ: -123.84 (s, 1F), -125.19 (s, 1F).

### Example A2: 2-(8-fluoro-3-quinolyl)-4,6,6-trimethyl-4-(2-methylallyl)-5H-1,3-oxazine (compound E.019).

### Step 1:

To a solution of acetonitrile (0.51 mL, 9.74 mmol) in diethyl ether (30 mL) at RT was added dropwise 2-methylallyl magnesium chloride (0.5 M in THF, 58 mL, 29.2 mmol, 3 equ.). The resulting solution was stirred at RT for 30 min, titanium isopropoxide (3.0 mL, 9.74 mmol, 1 equ.) was added dropwise and the solution was stirred for additional 5 h at RT. The reaction mixture was then slowly poured into a mixture of DCM (200 mL) NaOH (1M, 50 mL) and NaHCO₃ solution (50 mL). The resulting mixture was vigorously stirred for 20 min, filtered through celite and the phases were separated. The organic phase was washed with brine, dried over MgSO₄, filtered and concentrated *in vacuo* to give 2,4,6-trimethylhepta-1,6-dien-4-amine as a yellow liquid, which was used as such for the next step.

¹H NMR (400 MHz, CDCl₃) δ ppm: 4.70-5.06 (m, 4H), 2.11-2.20 (m, 4H), 1.79-1.91 (m, 6H), 1.01-1.17 (s, 3H).

### Step 2:

To a mixture of 8-fluoroquinoline-3-carboxylic acid (0.192 g, 1 mmol) and 2,4,6-trimethylhepta-1,6-dien-4-amine (0.20 g, 0.91 mmol) in DCM (7 mL) at RT was added triethylamine (0.32 mL, 2.28 mmol, 2.5 equ.), N-(3-Dimethylaminopropyl)-N'-ethylcarbodiimide hydrochloride (0.197 g, 1 mmol, 1.1 equ.) and 1-[bis(dimethylamino)methylene]-1H-1,2,3-triazolo[4,5-b]pyridinium 3-oxide hexafluorophosphate (0.394 g, 1 mmol, 1.1 equ.). The resulting solution was stirred for 1 h at RT and was then quenched with NaHCO₃ solution. The mixture was extracted with DCM, the organic phase was washed with brine, dried over MgSO₄, filtered and concentrated. The residue was purified by flash chromatography (silica gel, cyclohexane:EtOAc) to give N-[1,3-dimethyl-1-(2-methylallyl)but-3-enyl]-8-fluoro-quinoline-3-carboxamide as a white solid.

LC-MS (Method G), Rt = 1.11 min, (M+H) = 328.

¹H NMR (400 MHz, CDCl₃) δ ppm: 9.25 (d, 1H), 8.60 (s, 1H), 7.75 (d, 1H), 7.48-7.66 (m, 2H), 6.32 (br s, 1H), 4.76-5.08 (d, 4H), 2.91 (d, 2H), 2.53 (d, 2H), 1.88 (s, 6H), 1.51-1.66 (s, 3H).

### Step 3:

A solution of N-[1,3-dimethyl-1-(2-methylallyl)but-3-enyl]-8-fluoro-quinoline-3-carboxamide (0.03 g, 0.09 mmol) in acetonitrile (0.5 mL) at RT was treated with trifluoroacetic acid (0.1 mL), the mixture was warmed to 80°C and was stirred for 20 h at 80°C.The reaction mixture was cooled to RT and carefully poured on aq. NaHCO₃ solution. The neutralized mixture (pH > 8) was extracted with EtOAc, the combined organic layers were washed with brine, dried over MgSO₄, filtered and concentrated. The residue was purified by flash chromatography (silica gel, cyclohexane: EtOAc) to give 2-(8-fluoro-3-quinolyl)-4,6,6-trimethyl-4-(2-methylallyl)-5H-1,3-oxazine as yellow oil.

LC-MS (Method G), Rt = 1.03 min, (M+H) = 327.

¹H NMR (400 MHz, CDCl₃) δ ppm: 9.56 (d, 1H), 8.69 (br s, 1H), 7.71 (br d, 1H), 7.41-7.55 (m, 2H), 4.72-5.00 (m, 2H), 2.28-2.46 (m, 2H), 1.98-2.06 (m, 1H), 1.87-1.91 (m, 3H), 1.78-1.86 (m, 1H), 1.50 (s, 6H), 1.43 (s, 3H).

### Analytical Methods

### Method G:

Spectra were recorded on a Mass Spectrometer from Waters (SQD, SQDII Single quadrupole mass spectrometer) equipped with an electrospray source (Polarity: positive and negative ions), Capillary: 3.00 kV, Cone range: 30 V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment , diode-array detector and ELSD detector. Column: Waters UPLC HSS T3 , 1.8 µm, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 10-100% B in 1.2 min; Flow (ml/min) 0.85.

**Table E: Melting point (mp) and/or LC/MS data (retention time (Rt)) for compounds of Formula (I):**

| **Entry** | **IUPAC name** | **STRUCTURE** | **Rₜ (min)** | **[M+H] (measured)** | **Method** | **mp (°C)** |
|---|---|---|---|---|---|---|
| E.01 | 4-[[2-(8-fluoro-3-quinolyl)-4,6,6-trimethyl-5H-1,3-oxazin-4-yl]methoxy]benzonitrile | 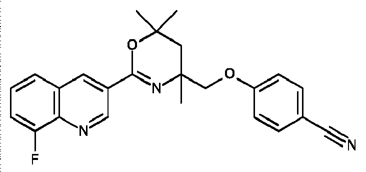 | 1.12 | 404 | G | |
| E.02 | 4-[(2,4-dibromophenoxy)methyl]-2-(8-fluoro-3-quinolyl)-4,6,6-trimethyl-5H-1,3-oxazine | 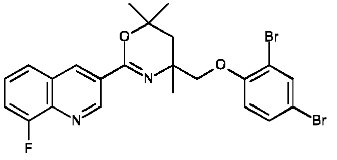 | 1.34 | 535 | G | |
| E.03 | 4-[(4-bromophenoxy)methyl]-2-(8-fluoro-3-quinolyl)-4,6,6-trimethyl-5H-1,3-oxazine | 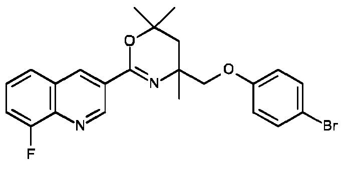 | 1.26 | 457 | G | |
| E.04 | 4-[(4-fluorophenoxy)methyl]-2-(8-fluoro-3-quinolyl)-4,6,6-trimethyl-5H-1,3-oxazine | 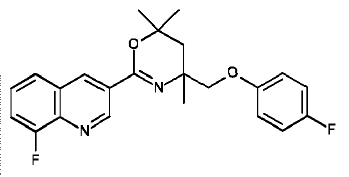 | 1.18 | 397 | G | |
| E.05 | 2-(7,8-difluoro-2-methyl-3-quinolyl)-4,6,6-trimethyl-4-(phenoxymethyl)-5H-1,3-oxazine | 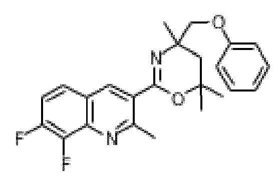 | 1.17 | 412 | G | |
| E.06 | 2-(7,8-difluoro-3-quinolyl)-4,6,6-trimethyl-4-(phenoxymethyl)-5H-1,3-oxazine | 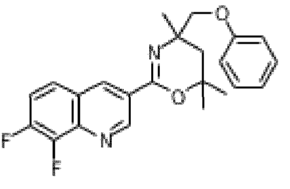 | 1.22 | 397 | G | 105 - 107 |
| E.07 | 2-(7,8-difluoro-3-quinolyl)-6,6-dimethyl-4-(phenoxymethyl)-4,5-dihydro-1,3-oxazine | 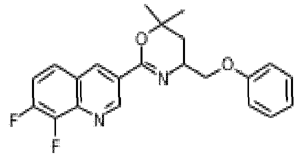 | 1.17 | 383 | G | |
| E.08 | 2-(8-fluoro-3-quinolyl)-4,6,6-trimethyl-4-(phenoxymethyl)-5H-1,3-oxazine | 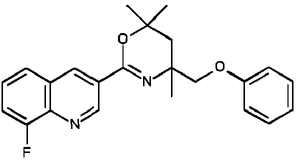 | 1.17 | 379 | G | |
| E.09 | 2-(7,8-difluoro-3-quinolyl)-6,6-dimethyl-4-(2,2,2-trifluoroethyl)-4,5-dihydro-1,3-oxazine | 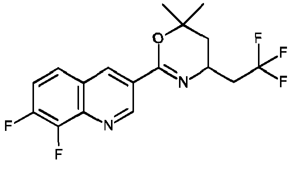 | 1.15 | 359 | | 146 - 148 |
| E.010 | 2-(7,8-difluoro-3-quinolyl)-6,6-dimethyl-4-(3,3,3-trifluoropropyl)-4,5-dihydro-1,3-oxazine | 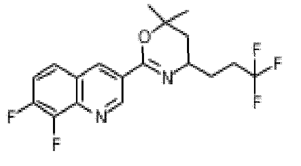 | 1.16 | 373 | G | |
| E.011 | 2-(7,8-difluoro-3-quinolyl)-4-(2,2-dimethylpropyl)-6,6-dimethyl-4,5-dihydro-1,3-oxazine | 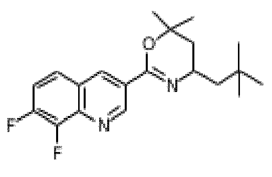 | 1.21 | 347 | G | 112 - 113 |
| E.012 | 2-(7,8-difluoro-3-quinolyl)-4-isobutyl-6,6-dimethyl-4,5-dihydro-1,3-oxazine | 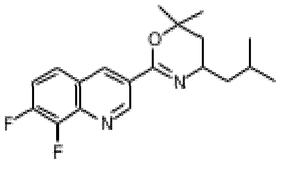 | 1.09 | 353 | G | 71 - 72 |
| E.013 | 2-(8-fluoro-3-quinolyl)-4-isobutyl-4,6,6-trimethyl-5H-1,3-oxazine | 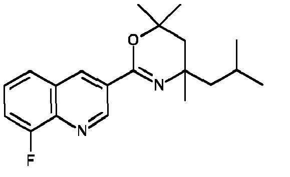 | 1.06 | 329 | G | |
| E.014 | 2-(7,8-difluoro-3-quinolyl)-4-isobutyl-4,6,6-trimethyl-5H-1,3-oxazine | 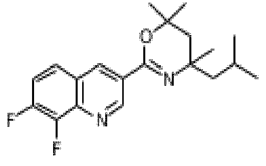 | 1.11 | 347 | G | |
| E.015 | 4-(cyclohexylmethyl)-2-(7,8-difluoro-3-quinolyl)-4,6,6-trimethyl-5H-1,3-oxazine | 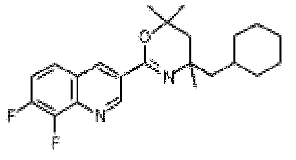 | 1.28 | 387 | G | |
| E.016 | 2-(7,8-difluoro-3-quinolyl)-4,6,6-trimethyl-4-(2-methylallyl)-5H-1,3-oxazine | 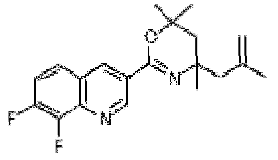 | 1.15 | 346 | G | |
| E.017 | 2-(8-fluoro-3-quinolyl)-4-isobutyl-6-isopropyl-4,6-dimethyl-5H-1,3-oxazine | 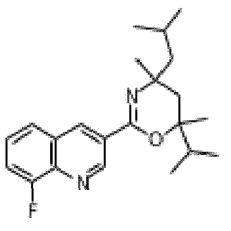 | 1.17 | 358 | G | |
| E.018 | 6-ethyl-2-(8-fluoro-3-quinolyl)-4-isobutyl-4,6-dimethyl-5H-1,3-oxazine | 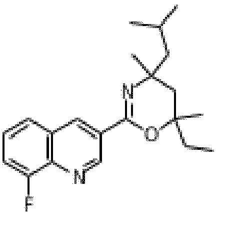 | 1.12 | 344 | G | |
| E.019 | 2-(8-fluoro-3-quinolyl)-4,6,6-trimethyl-4-(2-methylallyl)-5H-1,3-oxazine | 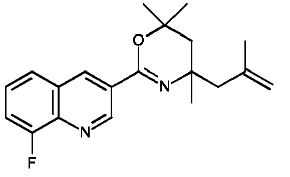 | 1.05 | 327 | G | |
| E.020 | 4-ethyl-2-(8-fluoro-3-quinolyl)-6,6-dimethyl-4-(2-methylallyl)-5H-1,3-oxazine | 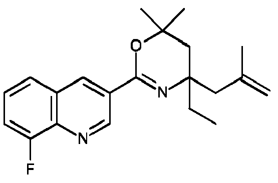 | 1.19 | 342 | G | |

### Biological Examples/Test Methods

### Botryotinia fuckeliana (Botrytis cinerea) / liquid culture (Gray mould)

Conidia of the fungus from cryogenic storage are directly mixed into nutrient broth (Vogels broth). After placing a (DMSO) solution of test compound into a microtiter plate (96-well format), the nutrient broth containing the fungal spores is added. The test plates are incubated at 24 °C and the inhibition of growth is determined photometrically 3-4 days after application.

The following compounds (from Table E) gave at least 80% control of *Botryotinia fuckeliana* at 20 ppm when compared to untreated control under the same conditions, which showed extensive disease development:
E.01, E.02, E.03, E.04, E.05, E.06, E.07, E.08, E.010, E.011, E.012, E.013, E.014, E.015, E.016, E.017, E.018, E.019, E.020.

### Glomerella lagenarium (Colletotrichum laqenarium) / liquid culture (Anthracnose)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is measured photometrically 3-4 days after application.

The following compounds (from Table E) gave at least 80% control of *Glomerella lagenarium* at 20 ppm when compared to untreated control under the same conditions, which showed extensive disease development:
E.03, E.04, E.08, E.012, E.013, E.014, E.015, E.016, E.017, E.018, E.019, E.020.

### Fusarium culmorum / liquid culture (Head blight)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 3-4 days after application.

The following compounds (from Table E) gave at least 80% control of *Fusarium culmorum* at 20 ppm when compared to untreated control under the same conditions, which showed extensive disease development:
E.04, E.06, E.07, E.08, E.014, E.015, E.018.

### Monographella nivalis (Microdochium nivale) / liquid culture (foot rot cereals)

Conidia of the fungus from cryogenic storage are directly mixed into nutrient broth (PDB potato dextrose broth). After placing a (DMSO) solution of test compound into a microtiter plate (96-well format), the nutrient broth containing the fungal spores is added. The test plates are incubated at 24 °C and the inhibition of growth is determined photometrically 4-5 days after application.

The following compounds (from Table E) gave at least 80% control of *Monographella nivalis* at 20 ppm when compared to untreated control under the same conditions, which showed extensive disease development:
E.01, E.02, E.03, E.04, E.05, E.06, E.07, E.08, E.09, E.010, E.011, E.012, E.013, E.014, E.015, E.016, E.017, E.018, E.019, E.020.

### Septoria tritici leaf spot on wheat / preventative

2-week old wheat plants cv. Riband are sprayed in a spray chamber with the formulated test compound diluted in water. The test plants are inoculated by spraying a spore suspension on them one day after application and then kept at 22°C/21°C (day/night) in a greenhouse. Disease damage is assessed directly when an appropriate level of disease appears on untreated check plants and efficacy was calculated compare to untreated controls (16 to 19 days after application).

The following compounds (from Table E) gave at least 80% control of *Septoria tritici* at 60 ppm when compared to untreated control under the same conditions, which showed extensive disease development:
E.025.

## Claims

1. A compound of formula (I): 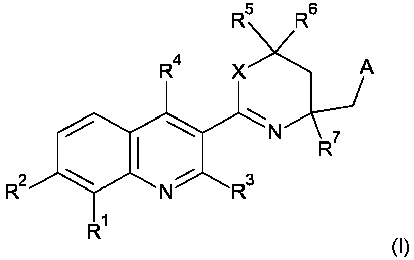 wherein:
X is O or S;
R¹ is halogen, methyl or cyano;
R² is hydrogen or halogen;
R³ and R⁴ are each independently hydrogen or methyl;
R⁵ and R⁶ are each independently selected from hydrogen, C₁-C₅alkyl, C₁-C₅haloalkyl, cyanoC₁₋Csalkyl, C₁-C₃alkoxyC₁-C₅alkyl, C₁-C₃alkylthioC₁-C₅alkyl, C₂-C₅alkenyl, C₂-C₅haloalkenyl, cyanoC₂-Csalkenyl, C₁-C₃alkoxyC₂-C₅alkenyl, C₁-C₃alkylthioC₂-C₅alkenyl, C₂-C₅alkynyl, C₂-C₅haloalkynyl, cyanoC₂-C₅alkynyl, C₁-C₃alkoxyC₂-C₅alkynyl, C₁-C₃alkylthioC₂-C₅alkynyl and C₃-C₆cycloalkyl, wherein cycloalkyl is optionally substituted with 1, 2 or 3 substituents independently selected from halogen, cyano, C₁-C₃alkyl, C₁-C₃alkoxy and C₁-C₃alkylthio; or
R⁵ and R⁶ together with the carbon atom to which they are attached represent C₃-C₆cycloalkyl, wherein the cycloalkyl group is optionally substituted with 1, 2 or 3 substituents independently selected from halogen, cyano and C₁-C₃alkyl;
R⁷ is hydrogen, C₁-C₄alkyl or C₃-C₄cycloalkyl;
A is C₁-C₅alkyl, C₂-C₅alkenyl, C₃-C₆cycloalkyl, C₄-C₆cycloalkenyl, C₁-C₅haloalkyl, C₂-Cshaloalkenyl, C₁-C₄alkoxy, C₃-C₅alkenyloxy, C₃-C₅alkynyloxy, C₁-C₄haloalkoxy, C₃-C₆cycloalkoxy and phenyloxy, wherein the C₃-C₆cycloalkoxy and phenyloxy is optionally substituted with 1, 2 or 3 substituents independently selected from R⁸;
R⁸ is halogen, nitro, cyano, C₁-C₅alkyl, C₁-C₂haloalkyl, C₁-C₅alkoxy, C₃-C₅alkenyloxy, C₃-Csalkynyloxy and C₁-C₅alkylthio; or
an agronomically acceptable salt, an N-oxide and/or S-oxide or a stereoisomer thereof.

2. The compound according to claim 1, wherein R¹ is fluoro.

3. The compound according to claim 1 or claim 2, wherein R² is hydrogen or fluoro.

4. The compound according to any one of claims 1 to 3, wherein R³ is methyl and R⁴ is hydrogen, R³ is hydrogen and R⁴ is methyl, or R³ and R⁴ are hydrogen.

5. The compound according to any one of claims 1 to 4, wherein R⁵ and R⁶ are each independently selected from hydrogen or C₁-C₅alkyl.

6. The compound according to any one of claims 1 to 4, wherein R⁵ and R⁶ together form a -(CH₂)ₙ- group bound to the carbon atom to which they are attached, wherein n is 2, 3, 4 or 5.

7. The compound according to any one of claims 1 to 6, wherein R⁷ is hydrogen, methyl, ethyl or iso-propyl.

8. The compound according to any one of claims 1 to 7, wherein A is selected from C₁-C₅alkyl, C₂-Csalkenyl, C₃-C₆cycloalkyl, C₄-C₆cycloalkenyl, C₁-C₄haloalkyl, C₂-C₅haloalkenyl, C₃-C₆cycloalkoxy and phenyloxy, wherein the C₃-C₆cycloalkoxy and phenyloxy is optionally substituted with 1 or 2 substituents independently selected from R⁸, wherein R⁸ is halogen, nitro, cyano, methyl, ethyl, trifluoromethyl, 2,2,2-trifluoroethyl, methoxy, ethoxy, C₃-C₅alkenyloxy, C₃-C₅alkynyloxy and C₁-C₃alkylthio.

9. The compound according to any one of claims 1 to 8, wherein A is selected from C₁-C₄alkyl, C₂-Csalkenyl, C₃-C₆cycloalkyl, C₄-C₆cycloalkenyl, C₁-C₄fluoroalkyl, C₂-C₅fluoroalkenyl, C₂-C₅chloroalkenyl, C₃-C₆cycloalkoxy and phenyloxy, wherein the C₃-C₆cycloalkoxy and phenyloxy is optionally substituted with 1 or 2 substituents independently selected from R⁸, wherein R⁸ is fluoro, chloro, bromo, nitro, cyano, methyl, ethyl, trifluoromethyl, methoxy and ethoxy.

10. The compound according to any one of claims 1 to 9, wherein A is selected from one of: 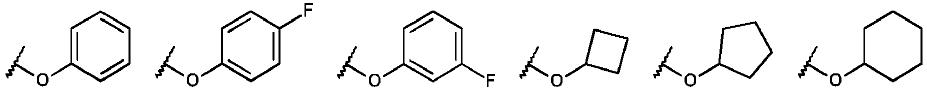 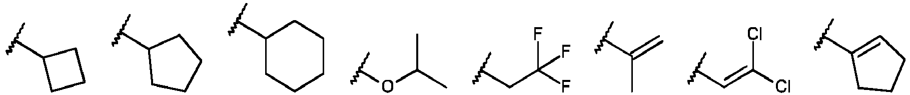 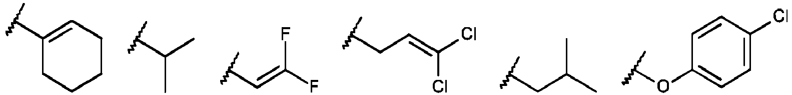 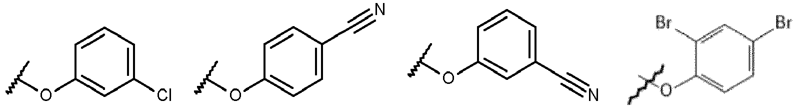 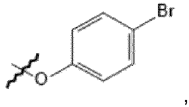 -CH₃, -CH₂CH₃, -CH₂CH₂CH₃, -C(CH₃)₃, -CF₃ and -CH₂CF₃.

11. The compound according to any one of claims 1 to 10, wherein X is O.

12. An agrochemical composition comprising a fungicidally effective amount of a compound according to any one of claims 1 to 11.

13. The composition according to claim 12, further comprising at least one additional active ingredient and/or an agrochemically-acceptable diluent or carrier.

14. A method of controlling or preventing infestation of useful plants by phytopathogenic microorganisms, wherein a fungicidally effective amount of a compound according to any of claims 1 to 11, or a composition comprising this compound as active ingredient according to claims 12 to 13, is applied to the plants, to parts thereof or the locus thereof.

15. Use of a compound according to any one of claims 1 to 11 as a fungicide, wherein the use excludes methods for the treatment of the human or animal body by surgery or therapy.

## Patentansprüche

1. Verbindung der Formel (I): 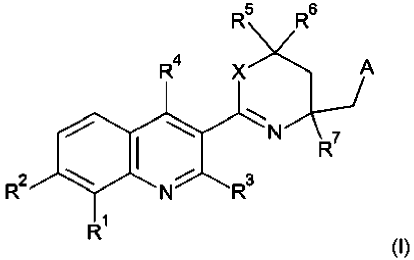 wobei:
X für 0 oder S steht,
R¹ für Halogen, Methyl oder Cyano steht;
R² für Wasserstoff oder Halogen steht,
R³ und R⁴ jeweils unabhängig für Wasserstoff oder Methyl stehen;
R⁵ und R⁶ jeweils unabhängig aus Wasserstoff, C₁-C₅-Alkyl, C₁-C₅-Halogenalkyl, Cyano-C₁-C₅-alkyl, C₁-C₃-Alkoxy-C₁-C₅-alkyl, C₁-C₃-Alkylthio-C₁-C₅-alkyl, C₂-C₅-Alkenyl, C₂-C₅-Halogenalkenyl, Cyano-C₂-C₅-alkenyl, C₁-C₃-Alkoxy-C₂-C₅-alkenyl, C₁-C₃-Alkylthio-C₂-C₅-alkenyl, C₂-C₅-Alkinyl, C₂-C₅-Halogenalkinyl, Cyano-C₂-C₅-alkinyl, C₁-C₃-Alkoxy-C₂-C₅-alkinyl, C₁-C₃-Alkylthio-C₂-C₅-alkinyl und C₃-C₆-Cycloalkyl ausgewählt sind, wobei Cycloalkyl gegebenenfalls durch 1, 2 oder 3 Substituenten, die unabhängig aus Halogen, Cyano, C₁-C₃-Alkyl, C₁-C₃-Alkoxy und C₁-C₃-Alkylthio ausgewählt sind, substituiert ist; oder
R⁵ und R⁶ zusammen mit dem Kohlenstoffatom, an das sie gebunden sind, für C₃-C₆-Cycloalkyl stehen, wobei die Cycloalkylgruppe gegebenenfalls durch 1, 2 oder 3 Substituenten, die unabhängig aus Halogen, Cyano und C₁-C₃-Alkyl ausgewählt sind, substituiert ist;
R⁷ für Wasserstoff, C₁-C₄-Alkyl oder C₃-C₄-Cycloalkyl steht;
A für C₁-C₅-Alkyl, C₂-C₅-Alkenyl, C₃-C₆-Cycloalkyl, C₄-C₆-Cycloalkenyl, C₁-C₅- Halogenalkyl, C₂-C₅-Halogenalkenyl, C₁-C₄-Alkoxy, C₃-C₅-Alkenyloxy, C₃-C₅-Alkinyloxy, C₁-C₄-Halogenalkoxy, C₃-C₆-Cycloalkoxy und Phenyloxy steht, wobei das C₃-C₆-Cycloalkoxy und Phenyloxy gegebenenfalls durch 1, 2 oder 3 Substituenten, die unabhängig aus R⁸ ausgewählt sind, substituiert sind;
R⁸ für Halogen, Nitro, Cyano, C₁-C₅-Alkyl, C₁-C₂-Halogenalkyl, C₁-C₅-Alkoxy, C₃-C₅-Alkenyloxy, C₃-C₅-Alkinyloxy und C₁-C₅-Alkylthio steht; oder
ein landwirtschaftlich unbedenkliches Salz, ein N-Oxid und/oder S-Oxid oder ein Stereoisomer davon.

2. Verbindung nach Anspruch 1, wobei R¹ für Fluor steht.

3. Verbindung nach Anspruch 1 oder Anspruch 2, wobei R² für Wasserstoff oder Fluor steht.

4. Verbindung nach einem der Ansprüche 1 bis 3, wobei R³ für Methyl steht und R⁴ für Wasserstoff steht, R³ für Wasserstoff steht und R⁴ für Methyl steht oder R³ und R⁴ für Wasserstoff stehen.

5. Verbindung nach einem der Ansprüche 1 bis 4, wobei R⁵ und R⁶ jeweils unabhängig aus Wasserstoff oder C₁-C₅-Alkyl ausgewählt sind.

6. Verbindung nach einem der Ansprüche 1 bis 4, wobei R⁵ und R⁶ zusammen eine an das Kohlenstoffatom, an das sie gebunden sind, gebundene -(CH₂)ₙ-Gruppe bilden, wobei n für 2, 3, 4 oder 5 steht.

7. Verbindung nach einem der Ansprüche 1 bis 6, wobei R⁷ für Wasserstoff, Methyl, Ethyl oder iso-Propyl steht.

8. Verbindung nach einem der Ansprüche 1 bis 7, wobei A aus C₁-C₅-Alkyl, C₂-C₅-Alkenyl, C₃-C₆-Cycloalkyl, C₄-C₆-Cycloalkenyl, C₁-C₄-Halogenalkyl, C₂-C₅-Halogenalkenyl, C₃-C₆-Cycloalkoxy und Phenyloxy ausgewählt ist, wobei das C₃-C₆-Cycloalkoxy und Phenyloxy gegebenenfalls durch 1 oder 2 Substituenten, die unabhängig aus R⁸ ausgewählt sind, substituiert sind, wobei R⁸ für Halogen, Nitro, Cyano, Methyl, Ethyl, Trifluormethyl, 2,2,2-Trifluorethyl, Methoxy, Ethoxy, C₃-C₅-Alkenyloxy, C₃-C₅-Alkinyloxy und C₁-C₃-Alkylthio ausgewählt sind.

9. Verbindung nach einem der Ansprüche 1 bis 8, wobei A aus C₁-C₄-Alkyl, C₂-C₅-Alkenyl, C₃-C₆-Cycloalkyl, C₄-C₆-Cycloalkenyl, C₁-C₄-Fluoralkyl, C₂-C₅-Fluoralkenyl, C₂-C₅-Chloralkenyl, C₃-C₆-Cycloalkoxy und Phenyloxy ausgewählt ist, wobei das C₃-C₆-Cycloalkoxy und Phenyloxy gegebenenfalls durch 1 oder 2 Substituenten, die unabhängig aus R⁸ ausgewählt sind, substituiert sind, wobei R⁸ für Fluor, Chlor, Brom, Nitro, Cyano, Methyl, Ethyl, Trifluormethyl, Methoxy und Ethoxy steht.

10. Verbindung nach einem der Ansprüche 1 bis 9, wobei A ausgewählt ist aus einem von: 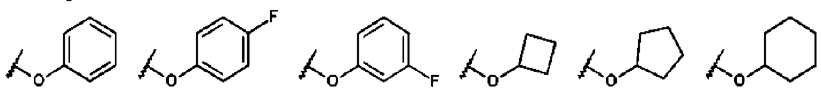 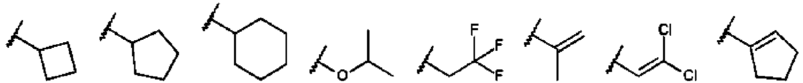 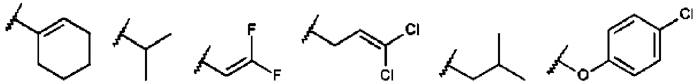 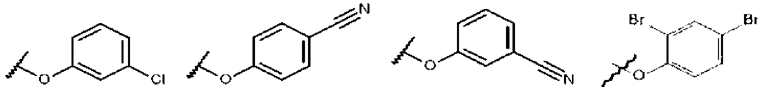 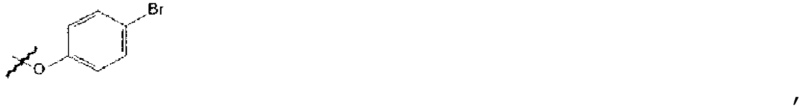 -CH₃, -CH₂CH₃, -CH₂CH₂CH₃, -C(CH₃)₃, -CF₃ und -CH₂CF₃.

11. Verbindung nach einem der Ansprüche 1 bis 10, wobei X für 0 steht.

12. Agrochemische Zusammensetzung, umfassend eine fungizid wirksame Menge einer Verbindung nach einem der Ansprüche 1 bis 11.

13. Zusammensetzung nach Anspruch 12, weiterhin umfassend mindestens einen zusätzlichen Wirkstoff und/oder ein agrochemisch unbedenkliches Verdünnungsmittel oder einen agrochemisch unbedenklichen Träger.

14. Verfahren zur Bekämpfung oder Prävention des Befalls von Nutzpflanzen durch phytopathogene Mikroorganismen, bei dem man eine fungizid wirksame Menge einer Verbindung nach einem der Ansprüche 1 bis 11 oder einer Zusammensetzung, die diese Verbindung als Wirkstoff umfasst, nach den Ansprüchen 12 bis 13
auf die Pflanzen, Teile
davon oder deren Standort ausbringt.

15. Verwendung einer Verbindung nach einem der Ansprüche 1 bis 11 als Fungizid, wobei die Verwendung Verfahren zur chirurgischen oder therapeutischen Behandlung des menschlichen oder tierischen Körpers ausschließt.

## Revendications

1. Composé de formule (I) : 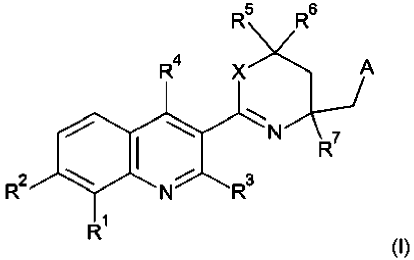
X étant 0 ou S ;
R¹ étant halogène, méthyle ou cyano ;
R² est hydrogène ou halogène ;
R³ et R⁴ étant chacun indépendamment hydrogène ou méthyle ;
R⁵ et R⁶ étant chacun indépendamment choisis parmi hydrogène, C₁-C₅alkyle, C₁-C₅halogénoalkyle, cyanoC₁-C₅alkyle, C₁₋C₃alcoxyC₁-C₅alkyle, C₁-C₃alkylthioC₁-C₅alkyle, C₂-C₅alcényle, C₂-C₅halogénoalcényle, cyanoC₂-C₅alcényle, C₁-C₃alcoxyC₂-C₅alcényle, C₁-C₃alkylthioC₂-C₅alcényle, C₂-C₃alcynyle, C₂-C₅halogénoalcynyle, cyanoC₂-C₅alcynyle, C₁-C₃alcoxyC₂-C₅alcynyle, C₁-C₃alkylthioC₂-C₅alcynyle et C₃-C₆cycloalkyle, cycloalkyle étant éventuellement substitué par 1, 2 ou 3 substituants indépendamment choisis parmi halogène, cyano, C₁-C₃alkyle, C₁-C₃alcoxy et C₁-C₃alkylthio ; ou
R⁵ et R⁶ conjointement avec l'atome de carbone auquel ils sont fixés représentant C₃-C₆cycloalkyle, le groupe cycloalkyle étant éventuellement substitué par 1, 2 ou 3 substituants indépendamment choisis parmi halogène, cyano et C₁-C₃alkyle ;
R⁷ étant hydrogène, C₁-C₄alkyle ou C₃-C₄cycloalkyle ;
A étant C₁-C₅alkyle, C₂-C₅alcényle, C₃-C₆cycloalkyle, C₄-C₆cycloalcényle, C₁-C₅halogénoalkyle, C₃-C₅halogénoalcényle, C₁-C₄alcoxy, C₃-C₅alcényloxy, C₃-C₅alcynyloxy, C₁-C₄halogénoalcoxy, C₃-C₆cycloalcoxy et phényloxy, les C₃-C₆cycloalcoxy et phényloxy étant éventuellement substitués par 1, 2 ou 3 substituants indépendamment choisis parmi R⁸ ;
R⁸ étant halogène, nitro, cyano, C₁-C₅alkyle, C₁-C₂halogénoalkyle, C₁-C₃alcoxy, C₃-C₅alcényloxy, C₃-C₅alcynyloxy et C₁-C₃alkylthio ; ou
sel acceptable sur le plan agronomique, N-oxyde et/ou S-oxyde ou stéréoisomère correspondant(s).

2. Composé selon la revendication 1, R¹ étant fluoro.

3. Composé selon la revendication 1 ou la revendication 2, R² étant hydrogène ou fluoro.

4. Composé selon l'une quelconque des revendications 1 à 3, R³ étant méthyle et R⁴ étant hydrogène, R³ étant hydrogène et R⁴ étant méthyle, ou R³ et R⁴ étant hydrogène.

5. Composé selon l'une quelconque des revendications 1 à 4, R⁵ et R⁶ étant chacun indépendamment choisis parmi hydrogène et C₁-C₅alkyle.

6. Composé selon l'une quelconque des revendications 1 à 4, R⁵ et R⁶ formant ensemble un groupe -(CH₂)ₙ- lié à l'atome de carbone auquel ils sont fixés, n étant 2, 3, 4 ou 5.

7. Composé selon l'une quelconque des revendications 1 à 6, R⁷ étant hydrogène, méthyle, éthyle ou iso-propyle.

8. Composé selon l'une quelconque des revendications 1 à 7, A étant choisi parmi C₁-C₅alkyle, C₂-C₅alcényle, C₃-C₆cycloalkyle, C₄-C₆cycloalcényle, C₁-C₄halogénoalkyle, C₂-C₅halogénoalcényle, C₃-C₆cycloalcoxy et phényloxy, les C₃-C₆cycloalcoxy et phényloxy étant éventuellement substitués par 1 ou 2 substituants indépendamment choisis parmi R⁸, R⁸ étant halogène, nitro, cyano, méthyle, éthyle, trifluorométhyle, 2,2,2-trifluoroéthyle, méthoxy, éthoxy, C₃-C₅alcényloxy, C₃-C₅alcynyloxy et C₁-C₃alkylthio.

9. Composé selon l'une quelconque des revendications 1 à 8, A étant choisi parmi C₁-C₄alkyle, C₂-C₅alcényle, C₃-C₆cycloalkyle, C₄-C₆cycloalcényle, C₁-C₄fluoroalkyle, C₂-C₅fluoroalcényle, C₂-C₅chloroalcényle, C₃-C₆cycloalcoxy et phényloxy, les C₃-C₆cycloalcoxy et phényloxy étant éventuellement substitués par 1 ou 2 substituants indépendamment choisis parmi R⁸, R⁸ étant fluoro, chloro, bromo, nitro, cyano, méthyle, éthyle, trifluorométhyle, méthoxy et éthoxy.

10. Composé selon l'une quelconque des revendications 1 à 9, A étant choisi parmi l'un parmi : 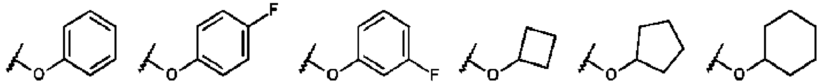 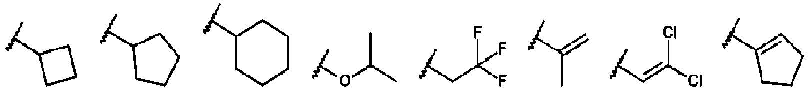 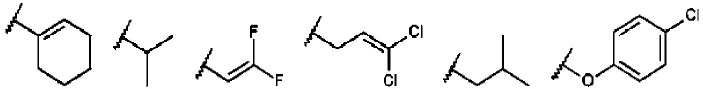 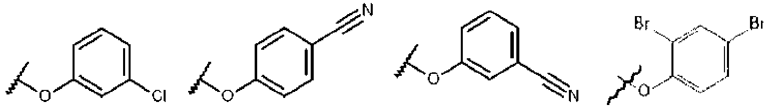 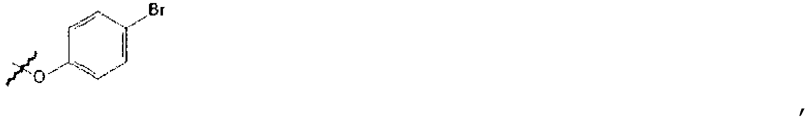 -CH₃, -CH₂CH₃, -CH₂CH₂CH₃, -C(CH₃)₃, -CF₃ et -CH₂CF₃.

11. Composé selon l'une quelconque des revendications 1 à 10, X étant O.

12. Composition agrochimique comprenant une quantité efficace sur le plan fongicide d'un composé selon l'une quelconque des revendications 1 à 11.

13. Composition selon la revendication 12, comprenant en outre au moins un ingrédient actif supplémentaire et/ou un diluant ou support acceptable sur le plan agrochimique.

14. Procédé de lutte contre ou de prévention d'une infestation de végétaux utiles par des micro-organismes phytopathogènes, une quantité efficace sur le plan fongicide d'un composé selon l'une quelconque des revendications 1 à 11, ou d'une composition comprenant ce composé en tant qu'ingrédient actif selon les revendications 12 à 13, étant appliquée sur les végétaux, sur des parties de ceux-ci ou le site de ceux-ci.

15. Utilisation d'un composé selon l'une quelconque des revendications 1 à 11 en tant que fongicide, l'utilisation excluant des procédés pour le traitement du corps humain ou animal par chirurgie ou thérapie.
